# EUROPÄISCHE PATENTSCHRIFT

(11) **EP 0 387 196 B1**
(45) Veröffentlichungstag und Bekanntmachung des Hinweises auf die Patenterteilung: **03.05.1995**
(21) Anmeldenummer: 90810152.0
(22) Anmeldetag: 27.02.1990
(51) Int. Cl.: C07F 7/00, C07F 7/08, C07F 7/28, C07C 29/00, C07C 209/00

(54) **Komplexe mit optisch aktiven Liganden, Verfahren zu deren Herstellung und deren Verwendung**
Complexes with optically active ligands, process for their preparation and their use
Complexes avec des ligands optiquement actifs, leur procédé de préparation et leur utilisation

(30) Priorität: 08.03.1989 CH 867/89; 28.09.1989 CH 3511/89
(43) Veröffentlichungstag der Anmeldung: 12.09.1990
(73) Patentinhaber: CIBA-GEIGY AG, 4002 Basel (CH)
(72) Erfinder: Hafner, Andreas, Dr., CH-4402 Frenkendorf (CH); Duthaler, Rudolf, Dr., CH-4126 Bettingen (CH); Bold, Guido, Dr., CH-5264 Gipf-Oberfrick (CH)

(56) Entgegenhaltungen:
- EP-A- 2 254 685
- Pure Appl. Chem. 55, 1983, 1807-1822
- Angew. Chemie, 95, 1983, 12-26
- HELVETICA CHIMICA ACTA, Band 64, Teil III, Heft 7, Nr. 243, 1981, Basel, Schweiz A.G. OLIVERO et al. "Enantio-selective addition of chiral organotitanium derivatives to aldehydes" Seiten 2485-2488
- CHEMISCHE BERICHTE, Band 118, 1985, Weinheim, D. SEEBACH et al. "Enantio-selective Addition von Aryl-gruppen an aromatische Aldehyde mit Aryltitan-Binaphtol-Derivaten" Seiten 3673-3682.

## Beschreibung

Die Erfindung betrifft Metallkomplexe mit Liganden von optisch aktiven 1,3-Dioxolan-4,5-dimethanolen, einem auf Carbonylgruppen oder carbonyl-analoge Gruppen übertragbaren Rest und einem Cyclopentadienylrest, ein Verfahren zu deren Herstellung und deren Verwendung als enantioselektive Reaktanden für Aldehyde, Ketone und N-substituierte Imine.

Aus Helvetica Chimica Acta, Vol. 64, Fasc. 7, No. 243, S. 2485-2488 (1981), Organometallics 3, S. 1716-1717 (1984) und Chem. Ber. 118, 3673-3682 (1985) ist bekannt, dass Titanverbindungen mit einer übertragbaren Gruppe und einem von einer chiralen Mono- oder Dihydroxyverbindung, zum Beispiel 1,3-Dioxolan-4,5-dimethanolen, abgeleiteten Rest mit Aldehyden zu sekundären Alkoholen umgesetzt werden können. Aliphatische chirale Hydroxyverbindungen als Liganden führen zu nur mässigen Stereoselektivitäten, während mit chiralem Binaphthol als Ligand hohe optische Ausbeuten erzielt werden können. Die verwendeten chiralen Liganden sind in technischen Mengen schwer zugänglich und teuer.

In der EP-A-0 254 685 sind Cyclopentadienyl-Titankomplexe mit optisch aktiven Zuckerliganden beschrieben, die bei der Umsetzung mit z.B. Aldehyden in hohen Ausbeuten bei hohen Enantioselektivitäten chirale sekundäre Alkohole ergeben. Auf Grund der natürlichen Zuckerliganden kann aber nur ein Enantiomer des Alkohols erhalten werden.

Es besteht ein Bedürfnis an preiswerten und in technischen Mengen verfügbaren chiralen Organometallreagenzien mit hohen Stereoselektivitäten bei der Reaktion mit prochiralen Aldehyden, Ketonen und Iminen, deren chiraler Ligand sich von einem der Enantiomeren- oder Diastereomerenpaare ableitet, so dass z.B. bei der Reaktion mit Aldehyden gezielt ein sekundärer Alkohol mit R- oder S-Konfiguration hergestellt werden kann.

Ein Gegenstand der Erfindung sind Verbindungen der Formel I 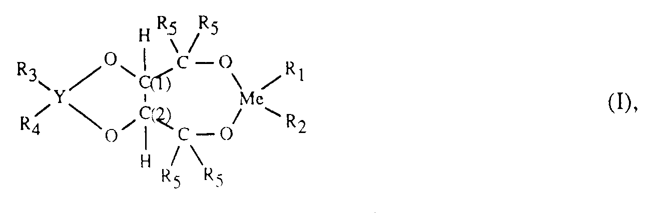
worin
a) Me für vierwertiges Titan steht und R₁ Allyl, 1-Methylallyl, 2-Methylallyl, -CH₂CH=CH-P(C₆H₅), -CH(SO₂R₆)CH=CH-Si(R₆)₃, 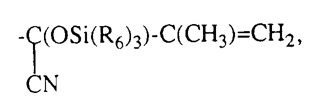 -CH₂-CH=CH-SO₂-R₆, -CH₂-CH=CH-OR' oder 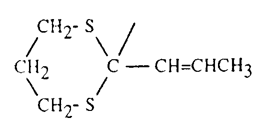 darstellt, R₆ für Phenyl, Benzyl oder C₁-C₈-Alkyl steht und R' C₁-C₈-Alkyl oder -Si(R₆)₃ bedeutet, oder R₁ einen über das O-Atom oder N-Atom gebundenen Rest eines Enols, Enamins oder Enhydrazins mit bis zu 20 C-Atomen bedeutet; oder
b) Me für vierwertiges Zirkonium oder Hafnium steht und R₁ lineares oder verzweigtes C₁-C₁₈-Alkyl, C₂-C₁₂-Alkenyl, C₂-C₁₂-Alkinyl, oder gegebenenfalls mit C₁-C₄-Alkyl substituiertes C₃-C₈-Cycloalkyl oder C₃-C₈-Cycloalkenyl, oder C₆-C₁₂-Aryl, C₇-C₁₆-Alkaryl, C₆-C₁₆-Aralkyl, C₈-C₁₆-Alkaralkyl, C₈-C₁₆-Aralkenyl, C₉-C₁₆-Alkaralkenyl, C₈-C₁₆-Aralkinyl oder C₉-C₁₆-Alkaralkinyl bedeutet, die unsubstituiert oder ein- oder mehrfach mit (C₆H₅)₂P-, (R₆O)₂P(O)-, (R₆)₃Si-, (R₆)₃SiO-, R₆SO₂-, -S-C₂-C₄-Alkylen-S-, -O-C₂-C₄-Alkylen-O-, wobei R₆ für Phenyl, Benzyl oder C₁-C₈-Alkyl steht, Cyano, F, Nitro, C₁-C₁₂-Alkylthio, C₁-C₁₂-Alkoxy, -NR₁₈R₁₉ oder -COR₇, wobei R₇ C₁-C₁₂-Alkoxy ist, R₁₈ und R₁₉ C₁-C₁₂-Alkyl, Cyclopentyl, Cyclohexyl, Phenyl, Benzyl oder (R₆)₃Si oder R₁₈ und R₁₉ zusammen C₃-C₆-Alkylen, 3-Oxapentylen oder -Si(R₆)₂-C₂-C₃-Alkylen-Si(R₆)₂- bedeuten, substituiert sind; oder R₁ einen über das O-Atom oder N-Atom gebundenen Rest eines Enols, Enamins oder Enhydrazins mit bis zu 20 C-Atomen bedeutet;
   R₂ gegebenenfalls durch C₁-C₆-Alkyl, C₂-C₆-Alkenyl, C₁-C₆-Alkoxy, C₅- oder C₆-Cycloalkyl, C₆-C₁₂-Aryl, C₇-C₁₃-Aralkyl, Tri-C₁-C₆-alkoxysilyl, Tri-C₁-C₆-alkylsilyl, R₆SO₂- oder Halogen substituiertes Cyclopentadienyl oder Indenyl darstellt,
   Y für C, Si oder -Si-O-SiR₃R₄- steht,
   R₃ und R₄ unabhängig voneinander H, lineares oder verzweigtes C₁-C₁₈-Alkyl, C₂-C₁₂-Alkenyl, oder gegebenenfalls mit C₁-C₄-Alkyl substituiertes C₃-C₈-Cycloalkyl oder C₃-C₈-Cycloalkenyl, oder C₆-C₁₂-Aryl, C₇-C₁₆-Aralkyl, C₇-C₁₆-Alkaryl, C₈-C₁₆-Alkaralkyl, C₈-C₁₆-Aralkenyl oder C₉-C₁₆-Alkaralkenyl oder R₃ und R₄ zusammen -CₙH₂ₙ- mit n gleich 3 bis 7 bedeuten, die unsubstituiert oder mit -CN, -F, -Cl, -Br, -Nitro, C₁-C₁₂-Alkylthio oder C₁-C₁₂-Alkoxy substituiert sind,
   R₅ unsubstituiertes oder mit C₁-C₁₂-Alkyl, C₁-C₁₂-Alkoxy, C₁-C₁₂-Alkylthio, Cyano, Di(C₁-C₄-Alkyl)amino, Di(C₁-C₄-Alkyl)aminocarbonyl, Fluor, C₆-C₁₀-Aryl, R₆SO₂-, C₁-C₁₂-Alkoxymethyl, C₁-C₁₂-Alkylthiomethyl, Di(C₁-C₄-Alkyl)aminomethyl oder Di(C₁-C₄-Alkyl)aminocarbonylmethyl substituiertes C₆-C₁₀-Aryl, Heteroaryl, Benzyl, Naphthylmethyl oder Heteroarylalkyl darstellt, wobei das Heteroaryl 5- oder 6-gliedrig ist und ein Heteroatom aus der Gruppe O, S und N enthält, oder C₁-C₁₂-Alkyl, C₂-C₁₂-Alkenyl oder -Alkinyl, C₃-C₈-Cycloalkyl oder -Cycloalkenyl bedeutet,
   und C(1) und C(2) chirale C-Atome sind, in Form von überwiegend Enantiomeren oder Diastereomeren.

Bedeutet Y ein C-Atom, so kann dieses ebenfalls chiral sein, wobei dann Diastereomere gebildet werden können.

R₁ ist eine auf Carbonyl- und Iminverbindungen übertragbare Gruppe.

R₁ als Alkyl enthält 1 bis 18, besonders 1 bis 12 und insbesondere 1 bis 6 C-Atome. Beispiele sind Methyl, Ethyl und die Isomeren von Propyl, Butyl, Pentyl, Hexyl, Heptyl, Octyl, Nonyl, Decyl, Undecyl, Dodecyl, Tridecyl, Tetradecyl, Pentadecyl, Hexadecyl, Heptadecyl und Octadecyl.

R₁ als Alkenyl enthält 2 bis 12, besonders 2 bis 6 C-Atome. Es kann sich sowohl um Alkenylalkyl der Formel 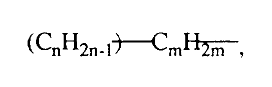
worin n eine Zahl von 2 bis 12, vorzugsweise 2 bis 6 und m eine Zahl von 1 bis 6, vorzugsweise 1 oder 2 sind, als auch um Alkylvinyl mit bevorzugt 1 bis 10 C-Atomen in der Alkylgruppe handeln. Beispiele sind: Allyl, 1-Methylallyl, 2-Methylallyl, But-2-en-4-yl, But-1-en-3-yl, Pent-3-en-5-yl, Pent-1-en-3-yl, Hex-4-en-6-yl, Hex-2-en-4-yl, Hept-2-en-1-yl, Hept-3-en-5-yl, Oct-6-en-8-yl, Oct-2-en-4-yl, Non-2-en-2-yl, Dec-8-en-10-yl, Dodec-3-en-12-yl, Vinyl, Crotonyl, n-But-1-en-1-yl, But-2-en-3-yl, Pent-1-en-2-yl, Hex-1-en-1-yl. Bevorzugt ist R₁ Vinyl oder Allyl. Besonders bevorzugt ist R₁ Allyl.

R₁ als Alkinyl enthält 2 bis 12, besonders 2 bis 6 C-Atome. Die Alkinylgruppe kann sich sowohl in der Kohlenstoffkette befinden oder endständig sein. Beispiele sind: Ethinyl, Prop-2-in-3-yl, Prop-1-in-3-yl, But-1-in-4-yl, But-3-in-4-yl, Pent-4-in-5-yl, Pent-3-in-5-yl, Pent-2-in-4-yl, Pent-1-in-5-yl, Hex-1-in-6-yl. Bevorzugt sind Ethinyl und Propargyl.

R₁ als Cycloalkyl oder Cycloalkenyl enthält 3 bis 8, besonders 3 bis 6 und insbesondere 5 oder 6 Ring-C-Atome, die mit C₁-C₄-Alkyl substituiert sein können. Beispiele sind Cyclopropyl, Cyclobutyl, Cyclopentyl, Cyclohexyl, Cycloheptyl, Cyclooctyl, Cyclodecyl, Cyclododecyl, Cyclopropenyl, Cyclobutenyl, Cyclopent-1-en-3-yl, Cyclopent-1-en-1-yl, Cyclohex-1-en-3-yl, Cyclohex-2-en-1-yl, Cyclohept-1-en-3-yl, Cyclooct-1-en-3-yl, 4-Methylcyclohex-1-en-3-yl.

R₁ als Aryl enthält 6 bis 12 C-Atome und ist bevorzugt Naphthyl und besonders Phenyl.

R₁ als Alkaryl enthält 7 bis 16 C-Atome. Das Aryl ist bevorzugt Phenyl. Beispiele sind: Methylphenyl, Dimethylphenyl, Ethylphenyl, n- und i-Propylphenyl, n-, i- und t-Butylphenyl, Di-t-Butylphenyl, Hexylphenyl, Octylphenyl, Decylphenyl.

R₁ als Aralkyl enthält 7 bis 16, besonders 7 bis 10 C-Atome. Das Aryl ist hierin bevorzugt Phenyl. Beispiele sind Benzyl, 1- oder 2-Phenylethyl, 1- oder 2-Phenylpropyl, Phenylbutyl, Phenylpentyl und Phenylhexyl. Bevorzugt sind Benzyl und 1- oder 2-Phenylethyl.

Als Alkaralkyl kann R₁ 8 bis 16 C-Atome enthalten. Das Aryl ist hierin bevorzugt Phenyl. Beispiele sind Methylbenzyl, Ethylbenzyl, n- oder i-Propylbenzyl, Dimethylbenzyl, n-, i- oder t-Butylbenzyl, Di-t-Butyl-benzyl, Hexylbenzyl, Nonylbenzyl, 1-Methylphenyleth-2-yl, 2-Methylphenyleth-2-yl, 1-Methylphenylprop-3-yl.

R₁ als Aralkenyl enthält 8 bis 16 C-Atome. Das Aryl ist hierin bevorzugt Phenyl. Beispiele sind Phenylvinyl, 1-Phenyl-prop-1-en-3-yl, 2-Phenyl-prop-2-en-1-yl, 3-Phenyl-prop-1-en-3-yl, Phenylbutenyl, Phenylpentenyl, Phenyhexenyl.

R₁ als Alkaralkenyl enthält 9 bis 16 C-Atome. Das Aryl ist hierin bevorzugt Phenyl. Beispiele sind Methylphenylvinyl, Ethylphenylvinyl, Dimethylphenylvinyl, 1-Methylphenyl-prop-2-en-3-yl, 2-Methylphenyl-prop-2-en-3-yl.

R₁ als Aralkinyl enthält 8 bis 16 C-Atome. Das Aryl ist hierin bevorzugt Phenyl. Beispiele sind Phenylethinyl, Phenylpropinyl, 1-Phenylbut-3-in-4-yl.

R₁ als Alkaralkinyl enthält 9 bis 16 C-Atome. Das Aryl ist hierin bevorzugt Phenyl. Beispiele sind Methylphenylethinyl und Methylphenylpropargyl.

Einige Beispiele für Reste R₁ sind -CH₂P(O)(OR₆)₂, Methyl, Ethyl, Vinyl, Allyl, Crotyl und substituierte Allylreste wie z.B. -CH₂CH=CH-P(C₆H₅), -CH(SO₂R₆)CH=CH-Si(R₆)₃, 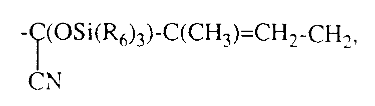
-CH=CH-SO₂-R₆, -CH₂-CH=CH-OR' und 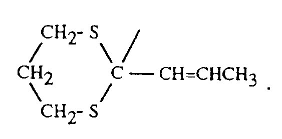
R₆ kann die vorher angegebene Bedeutung haben. Bei R' kann es sich beispielsweise um C₁-C₈-Alkyl oder -Si(R₆)₃ handeln.

Der Enolrest R₁ ist über das Enolsauerstoffatom an das Metall Me gebunden. Es kann sich um lineare oder cyclische Enole handeln. Der Enolrest R₁ enthält bis zu 20, besonders 2 bis 16 C-Atome. Die cyclischen Enole können 3 bis 8, besonders 4 bis 6 Ringatome enthalten, wobei der Ring aus Atomen der Gruppe C, O, S und N gebildet sein kann. Der Enolrest R₁ kann z.B. der allgemeinen Formel 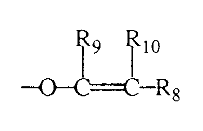
entsprechen. R₉ kann z.B. H, gegebenenfalls substituiertes C₁-C₈-Alkyl, C₁-C₈-Alkoxy, C₁-C₈-Alkylthio, Cycloalkyl mit 3 bis 6 Ring-C-Atomen Phenyl, Benzyl, Phenoxy, Phenylthio, Benzyloxy, Benzylthio, R₁₈R₁₉N-, -OB(OC₁-C₈-Alkyl)₂, -OSn(R₁₁)₃ mit R₁₁ gleich C₁-C₈-Alkyl, Phenyl oder Benzyl, -OTi(R₁₁)₃, -OLi, -OSi(R₆)₃ sein. R₁₀ und R₈ können unabhängig für H, F oder gegebenenfalls substituiertes C₁-C₈-Alkyl, C₁-C₈-Alkoxy, C₁-C₈-Alkylthio, Cycloalkyl mit 3 bis 6 Ring-C-Atomen, Phenyl, Benzyl, Phenoxy, Phenylthio, Benzyloxy, Benzylthio, R₁₈R₁₉N- oder -OSi(R₆)₃ stehen. Als Substituenten für R₉ kommen die für R₁ genannten Substituenten in Frage. Das Sekundäramino kann C₁-C₈-Alkylgruppen, Phenyl, Benzyl, oder ((R₆)₃)Si-Gruppen enthalten. Es kann sich auch um heterocyclische Sekundäraminogruppen handeln, die vorzugsweise 4 bis 6 Ringatome aus der Gruppe C, O, S, Si und N enthalten. R₉ und R₁₀ bzw. R₁₀ und R₈ können zusammen mit den C-Atomen, an die sie gebunden sind, einen 3- bis 8-, vorzugsweise 4- bis 6-gliedrigen carbocyclischen oder heterocyclischen Ring bilden, wobei der heterocyclische Ring Atome aus der Gruppe C, O, S und N enthalten kann. Die Ringe können wie zuvor für R₁ definiert substituiert sein. Eine bevorzugte Gruppe sind Esterenolate, worin R₉ C₁-C₈-Alkoxy bedeutet. Eine andere bevorzugte Gruppe sind Enole von Glycinderivaten, die z.B. der Formel 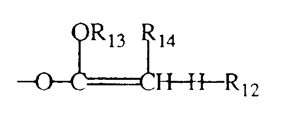
entsprechen. R₁₃ kann z.B. Benzyl, Phenyl oder C₁-C₈-Alkyl sein. R₁₄ und R₁₂ können C₁-C₈-Alkyl, Phenyl oder Benzyl sein, oder sie können zusammen mit dem N-Atom einen gegebenenfalls weitere Heteroatome aus der Gruppe O, S oder N enthaltenden Heterocyclus bilden. R₁₃ und R₁₄ zusammen können auch Methylen oder Ethylen darstellen. R₁₄ und R₁₂ können auch abspaltbare Schutzgruppen sein, z.B. (R₆)₃Si- oder -Si(R₆)₂CH₂CH₂Si(R₆)₂-. R₁₃, R₁₄ und R₁₂ können wie zuvor für R₁ definiert substituiert sein. Eine andere bevorzugte Gruppe sind Enolreste der Formel 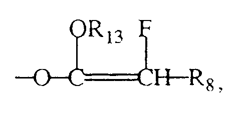
worin R₈ und R₁₃ die zuvor angegebene Bedeutung haben.

Bei R₁ kann es sich auch um den Rest eines Enamins oder Enhydrazins handeln, die über ein N-Atom an das Metall Me gebunden sind. Es kann sich um lineare oder cyclische Enamine bzw. Enhydrazine handeln, die bis zu 20, besonders 2 bis 16 C-Atome enthalten. Die cyclischen Enamine und Enhydrazine können 3 bis 8, besonders 4 bis 6 Ringatome enthalten, wobei der Ring aus Atomen der Gruppe C, O, S und N gebildet sein kann. Das N-Atom der Enamingruppe kann substituiert sein, bevorzugt mit C₁-C₈-Alkyl, Phenyl, Benzyl oder mit Schutzgruppen wie z.B. (R₆)₃Si-.

Der Enaminrest bzw. Enhydrazinrest kann z.B. der Formel 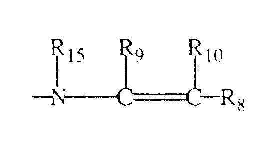
entsprechen, worin R₉, R₁₀ und R₈ die zuvor für den Enolrest R₁ angegebene Bedeutung haben, R₉ und R₁₅ zusammen mit der C-N-Gruppe oder R₁₀ und R₁₅ zusammen mit der -N-C=C-Gruppe einen 3- bis 8-, vorzugsweise 4- bis 6-gliedrigen Heterocyclus bilden, der weitere Heteroatome aus der Gruppe 0, S und N enthalten kann, und R₁₅ H, C₁-C₈-Alkyl, Phenyl, Benzyl, C₁-C₈-Alkoxy, Phenoxy, Benzyloxy, (R₆)₃Si- oder -NR₁₆R₁₇ bedeutet, worin R₆ die zuvor angegebene Bedeutung hat und R₁₆ und R₁₇ H, C₁-C₈-Alkyl, Phenyl oder Benzyl sind. In einer bevorzugten Gruppe sind R₉ H, C₁-C₈-Alkyl, Phenyl oder Benzyl und R₁₀ und R₈ H. Die Enol-, Enamin- und Enhydrazinreste sind C-Nukleophile, die zu Aldolreaktionen befähigt sind.

R₁ als Rest eines Enols kann auch ein Ketonenolat sein das besonders bis zu 16, insbesondere 4 bis 16 C-Atome enthält. Diese Enolate können sich ableiten von β-Ketoaldehyden, 1,3-Diketonen, β-Ketocarbonsäureestern und β-Ketocarbonsäureamiden. Die Estergruppe enthält bevorzugt Reste von aliphatischen C₁-C₆-Alkoholen. Das N-Atom in der Amidgruppe kann 1 oder 2-fach substituiert sein, bevorzugt mit C₁-C₆-Alkyl. Beispiele sind: β-Ketobutyraldehyd, Acetylaceton, Benzoylaceton, Acetylessigsäureethylester und Acetylessigsäureamid.

Der Rest R₁ kann ein- oder mehrfach, bevorzugt ein- bis dreifach, besonders ein- oder zweifach substituiert sein. Ist der Substituent Alkoxy oder Alkylthio, so enthält dieser bevorzugt 1 bis 6 C-Atome. R₇ ist bevorzugt der Rest eines aliphatischen Alkohols mit 1 bis 6 C-Atomen. Es kann sich z.B. um Reste aliphatischer oder cycloaliphatischer Alkohole handeln. Beispiele sind Methoxy, Ethoxy, n-und i-Propoxy, n-, i- und t-Butoxy, Pentoxy, Hexyloxy, Cyclopentyloxy und Cyclohexyloxy.

R₆ enthält als Alkyl bevorzugt 1 bis 6, besonders 1 bis 4 C-Atome. Das Alkoxy, Alkylthio, -S-C₂-C₄-Alkylen-S- und -O-C₂-C₄-Alkylen-O- können an ein C-Atom gebunden sein; es handelt sich dann um Acetal-oder Ketalgruppen.

Das Sekundäramino in der Bedeutung als Substituent entspricht der Formel -NR₁₈R₁₉, worin R₁₈ und R₁₉ C₁-C₁₂-, bevorzugt C₁-C₆-Alkyl, Cyclopentyl, Cyclohexyl, Phenyl, Benzyl oder (R₆)₃Si- sind, oder R₁₈ und R₁₉ zusammen C₃-C₆-Alkylen, 3-Oxapentylen oder -Si(R₆)₂-C₂-C₃-Alkylen-Si(R₆)₂- bedeuten und R₆ die zuvor angegebene Bedeutung hat.

In einer bevorzugten Untergruppe ist R₁ gegebenenfalls ein- oder mehrfach mit -NR₁₈R₁₉ Cyano, Nitro, C₁-C₆-Alkylthio, C₁-C₆-Alkoxy oder -COR₇, worin R₇ C₁-C₁₂-Alkoxy ist, substituiertes lineares oder verzweigtes C₁-C₁₂-Alkyl, C₂-C₁₂-Alkenyl, C₂-C₁₂-Alkinyl, Cycloalkyl oder Cycloalkenyl mit 3 bis 8 Ring-C-Atomen, C₆-C₁₂-Aryl, C₇-C₁₆-Alkaryl oder -Aralkyl, C₈-C₁₆-Alkaralkyl, C₈-C₁₆-Aralkenyl, C₉-C₁₆-Alkaralkenyl, C₈-C₁₆-Aralkinyl oder C₉-C₁₆-Alkaralkinyl; oder ein über das Enolsauerstoffatom oder über das Enaminstickstoffatom gebundener Rest eine Enols, Enamins oder Enhydrazins.

In einer weiteren bevorzugten Untergruppe ist R₁ gegebenenfalls ein- oder mehrfach mit -NR₁₈R₁₉, Cyano, Nitro, C₁-C₆-Alkoxy, C₁-C₆-Alkylthio oder -COR₇, worin R₇ C₁-C₁₂-Alkoxy ist, substituiertes lineares oder verzweigtes C₁-C₆-Alkyl, C₂-C₆-Alkenyl, C₂-C₆-Alkinyl, Cycloalkyl oder Cycloalkenyl mit 3 bis 6 Ring-C-Atomen, Phenyl, (C₁-C₁₀-Alkyl)phenyl, Phenyl(C₁-C₂-alkyl), (C₁-C₈-Alkyl)phenyl(C₁-C₂-alkyl), Phenylvinyl, Phenylethinyl oder Phenylpropargyl, (C₁-C₈-Alkyl)phenylvinyl, (C₁-C₈-Alkyl)phenylethinyl oder (C₁-C₇-Alkyl)phenylpropargyl; oder ein über ein Enolsauerstoffatom oder Enaminstickstoffatom gebundener Rest eines Enols, Enamins oder Enhydrazins mit bis zu 20 C-Atomen.

Besonders bevorzugt ist R₁ gegebenenfalls ein- oder mehrfach mit -NR₁₈R₁₉, Cyano, Nitro, C₁-C₆-Alkoxy, C₁-C₆-Alkylthio oder -COR₇, worin R₇ C₁-C₁₂-Alkoxy ist, substituiertes Methyl, Ethyl, Vinyl, Allyl, Crotonyl, Ethinyl, Propargyl, Cyclopentyl, Cyclohexyl, Cyclopentenyl oder Cyclohexenyl, Phenyl, Methylphenyl, Benzyl, 1-Phenyleth-2-yl, Methylbenzyl, Phenylvinyl, Methylphenylvinyl, Phenylethinyl, Phenylpropargyl, Methylphenylethinyl, Dimethylphenylethinyl oder -propargyl; oder ein über das Enolsauerstoffatom oder über das Enaminostickstoffatom gebundener Rest eines Enols, Enamins oder Enhydrazins mit 2 bis 16 C-Atomen.

Insbesondere stellt R₁ Methyl, Ethyl, Allyl oder den Rest eines Esterenolats dar, das mit Sekundäramino substituiert sein kann.

R₂ in der Bedeutung von Cyclopentadienyl oder Indenyl kann substituiert sein, beispielsweise mit 1 bis 5, zum Beispiel 1 oder 2 Substituenten. Geeignete Substituenten sind zum Beispiel Alkyl mit bevorzugt 1 bis 6 C-Atomen, Alkenyl mit bevorzugt 2 bis 6 C-Atomen, Alkoxy mit bevorzugt 1 bis 6 C-Atomen, Cycloalkyl mit bevorzugt 5 oder 6- Ring-C-Atomen, Aryl mit bevorzugt 6 bis 12 C-Atomen, Aralkyl mit bevorzugt 7 bis 13 C-Atomen, Trialkoxysilyl mit bevorzugt 1 bis 6 C-Atomen in den Alkoxylgruppen, Trialkylsilyl mit bevorzugt 1 bis 6 C-Atomen in Alkylgruppen, Halogen, bevorzugt F, Cl und Br oder R₆SO₂- mit R₆ bevorzugt gleich Phenyl Benzyl oder C₁-C₆-Alkyl. Aryl ist besonders Phenyl, und Aralkyl ist besonders Benzyl.

Wenn Me für Titan steht, enthält R₂ in der Bedeutung von Cyclopentadienyl oder Indenyl bevorzugt keinen, oder 1 bis 5, besonders 1, 2 oder 3 Substituenten. Wenn Me für Zirkonium oder Hafnium steht, enthält R₂ in der Bedeutung von Cyclopentadienyl oder Indenyl bevorzugt 3 bis 5 Substituenten, besonders Methyl- oder Trialkylsilylgruppen.

In einer bevorzugten Ausführungsform bedeutet R₂ gegebenenfalls mit C₁-C₆-Alkyl, C₂-C₆-Alkenyl, C₁-C₆-Alkoxy, Cycloalkyl mit 5 oder 6 Ring- C-Atomen, Phenyl, Benzyl, Trialkoxysilyl mit 1 bis 6 C-Atomen in den Alkoxygruppen, Trialkylsilyl mit 1 bis 6 C-Atomen in den Alkylgruppen, F, Cl, Br, oder R₆SO₂- substituiertes Cyclopentadienyl oder Indenyl.

In einer anderen bevorzugten Ausführungsform bedeutet R₂ gegebenenfalls mit C₁-C₆-Alkyl oder Trimethylsilyl substituiertes Cyclopentadienyl.

Besonders bevorzugt ist R₂ Cyclopentadienyl, Pentamethylcyclopentadienyl, Trimethylsilylcyclopentadienyl, Bis- oder Tri(trimethylsilyl)cyclopentadienyl.

Für R₃ und R₄ in der Bedeutung als Kohlenwasserstoffreste gelten die gleichen Bevorzugungen wie für R₁. Beispiele sind für R₁ aufgezählt worden. R₃ und R₄ enthalten als Alkyl 1 bis 18, besonders 1 bis 12 und insbesondere 1 bis 6 C-Atome. Beispiele sind Methyl, Ethyl, n- oder i-Propyl, n-, i-oder t-Butyl, Pentyl, Hexyl, Heptyl, Octyl, Nonyl, Decyl, Undecyl, Dodecyl, Tetradecyl, Hexadecyl, Octadecyl und Eicosyl. Besonders bevorzugt sind Methyl, Ethyl, Propyl und Butyl. R₃ und R₄ enthalten als Cycloalkyl bevorzugt 5 oder 6 Ring-C-Atome. Beispiele sind Cyclopentyl, Cyclohexyl und Methylcyclohexyl. R₃ und R₄ zusammen als Gruppe -CₙH₂ₙ- bedeuten lineares oder verzweigtes Alkylen mit 3 bis 7, bevorzugt 4 bis 6 C-Atomen in der Alkylengruppe. Besonders bevorzugte Substituenten für R₃ und R₄ sind -F, -Cl, C₁-C₆-Alkylthio oder C₁-C₆-Alkoxy.

In einer bevorzugten Ausführungsform sind R₃ und R₄ unabhängig voneinander H, C₁-C₁₂-Alkyl, C₂-C₁₂-Alkenyl, gegebenenfalls mit C₁-C₄-Alkyl substituiertes Cycloalkyl oder Cycloalkenyl mit 5 oder 6 Ring-C-Atomen, Phenyl, Naphthyl, Benzyl, C₁-C₆-Alkylphenyl oder C₁-C₆-Alkylbenzyl oder R₃ und R₄ zusammen -CₙH₂ₙ- mit n gleich 4 bis 6, die unsubstituiert oder mit -CN, -F, -Cl, -Nitro, C₁-C₆-Alkylthio oder C₁-C₆-Alkoxy substituiert sind.

In einer anderen bevorzugten Ausführungsform sind R₃ und R₄ unabhängig voneinander H, C₁-C₆-Alkyl, z.B. Methyl, Ethyl, Propyl und Butyl, gegebenenfalls mit C₁-C₄-Alkyl substituiertes Cyclopentyl oder Cyclohexyl, Phenyl, Benzyl, C₁-C₆-Alkylphenyl oder C₁-C₆-Alkylbenzyl oder R₃ und R₄ zusammen Penta- oder Tetramethylen.

Eine bevorzugte Untergruppe sind solche Verbindungen, worin R₃ H und R₄ oder R₃ und R₄ einer der zuvor definierten Kohlenwasserstoffreste sind.

Y in Formel I steht besonders für C. Wenn dieses C-Atom chiral ist, so umfassen die Verbindungen der Formel I Diastereomere. R₃R₄Y kann R₃R₄Si sein, worin R₃ und R₄ bevorzugt C₁-C₆-Alkyl besonders C₁-C₄-Alkyl darstellen. R₃R₄Y kann auch -SiR₃R₄-O-SiR₃R₄- sein, worin R₃ und R₄ bevorzugt C₁-C₄-Alkyl bedeuten.

R₅ stellt als Aryl C₆-C₁₀-Aryl dar und ist besonders Phenyl oder Naphthyl. Als Heteroaryl stellt R₅ fünf- oder 6-gliedriges Heteroaryl mit einem Heteroatom aus der Gruppe O, S und N dar. R₅ stellt als Aralkyl Benzyl oder Naphthylmethyl und Heteroarylmethyl dar, wobei das Heteroaryl 5 oder 6 Ringglieder enthält und Heteroatome aus der Gruppe O, S und N. Beispiele für Heteroaryl sind Pyridyl, Furyl, Thiophenyl und Pyrryl. Die Substituenten Alkyl, Alkoxy und Alkylthio enthalten bevorzugt 1 bis 6 C-Atome.

R₅ kann als Alkyl, Alkenyl und Alkinyl den für R₁ angegebenen Bevorzugungen entsprechen.

R₅ bedeutet bevorzugt C₂-C₄-Alkenyl, 2-Furyl oder C₆-C₁₀-Aryl, das unsubstituiert oder mit C₁-C₆-Alkyl oder F substituiert ist. Das Aryl ist bevorzugt Methylphenyl, Pentafluorphenyl, Phenyl oder Naphthyl. R₅ bedeutet besonders Propenyl, Phenyl, Pentafluorphenyl, Methylphenyl oder Furyl.

Eine bevorzugte Ausführungsform sind Verbindungen der Formel I, worin
R₁ lineares oder verzweigtes C₁-C₄-Alkyl, C₂-C₆-Alkenyl, oder gegebenenfalls mit C₁-C₄-Alkyl substituiertes Cycloalkyl oder Cycloalkenyl mit 3 bis 6 Ring-C-Atomen, oder Phenyl, Benzyl, C₁-C₆-Alkylphenyl oder C₁-C₆-Alkylbenzyl darstellt, das unsubstituiert oder mit Sekundäramino, Cyano, Nitro, C₁-C₆-Alkoxy, C₁-C₆-Alkylthio oder -COR₇, worin R₇ C₁-C₁₂-Alkoxy ist, darstellt, oder R₁ einen über ein Enolsauerstoffatom oder ein Enaminostickstoffatom gebundener Rest eines Enols, Enamins oder Enhydrazins mit bis zu 12 C-Atomen bedeutet,
R₂ Cyclopentadienyl ist, das unsubstituiert oder mit C₁-C₄-Alkyl oder Trimethylsilyl substituiert ist,
Y für C steht,
R₃ H ist oder die Bedeutung von R₄ hat und
R₄ C₁-C₁₂-Alkyl, Phenyl, Benzyl, Cyclopentyl oder Cyclohexyl darstellt oder
R₃ und R₄ zusammen Tetra- oder Pentanmethylen bedeuten,
R₅ für C₂-C₄-Alkenyl, Phenyl, Pentafluorphenyl oder Furyl steht, und
Me vierwertiges Titan, Zirkonium oder Hafnium bedeutet.

Die C(1)- und C(2)-Atome in Formel I weisen bevorzugt R,R- oder S,S-Konfiguration auf. Stellt Y in Formel I ein chirales C-Atom dar, so können die C(1)- und C(2)-Atome auch in R,S- oder S,R-Konfiguration vorliegen.

Eine besonders bevorzugte Ausführungsform sind Verbindungen der Formel I, worin R₁ C₁-C₄-Alkyl, Allyl oder der Rest eines Esterenolats mit 2 bis 12 C-Atomen ist, das mit Sekundäramino substituiert sein kann, R₂ gegebenenfalls mit Methyl oder Trimethylsilyl substituiertes Cyclopentadienyl bedeutet, R₃ H darstellt oder die Bedeutung von R₄ hat und R₄ C₁-C₆-Alkyl ist, R₅ Propenyl, Phenyl oder Pentafluorphenyl oder Furyl bedeutet, Me für vierwertiges Titan, Zirkonium, Hafnium und Y für C stehen.

Die Verbindungen der Formel I können hergestellt werden, indem man eine Verbindung der Formel II 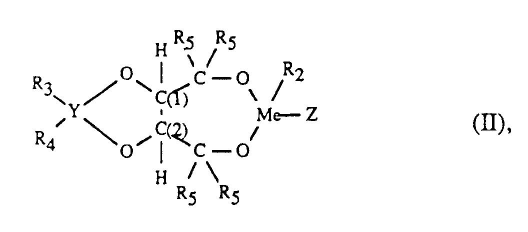
mit einer Verbindung der Formel III

R₁^{⊖}-M^{⊕} (III)

in Gegenwart eines inerten Lösungsmittels und inerten Schutzgases umsetzt, wobei R₁, R₂, R₃, R₄, R₅, Me und Y die oben angegebenen Bedeutungen haben, Z für ein Anion steht und M^{⊕} für Li^{⊕}, Na^{⊕}, K^{⊕}, MgX^{⊕}, ZnX^{⊕}, CdX^{⊕}, HgX^{⊕}, CuX^{⊕} oder quaternäres Ammonium steht, wobei X Halogen bedeutet.

In der Gruppe M^{⊕} der Formel III steht X bevorzugt für Cl, Br oder I. M^{⊕} bedeutet bevorzugt Li^{⊕}, MgCl^{⊕}, MgBr^{⊕}, ZnCl^{⊕}, ZnBr^{⊕}, CdCl^{⊕}, CdBr^{⊕} oder Tetraalkylammonium mit 1 bis 6 C-Atomen in den Alkylgruppen. Besonders bevorzugt bedeutet M^{⊕} MgX^{⊕} und Li^{⊕}, worin X Cl, Br oder I ist.

Als Anion Z kommen z.B. PF₆^{⊖}-, SbF₆^{⊖}-, BF₄^{⊖}-, CF₃COO^{⊖}, Sulfonat (z.B. Tosylat), und besonders Cl^{⊖} oder Br^{⊖} in Frage.

Die Verbindungen der Formel II sind ein weiterer Gegenstand der Erfindung. Sie sind in einfacher Weise durch die Umsetzung von 1 Mol eines Salzes der Formel

(R₂)MeZ₃

mit 1 Mol einer Verbindung der Formel IV 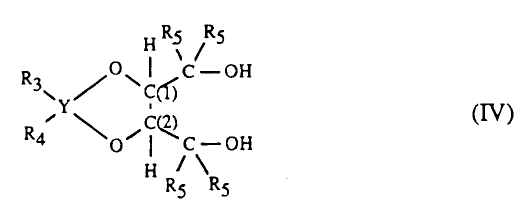
erhältlich. Die Verbindungen der Formel IV sind aus entsprechend optisch aktiven und mit der Gruppe R₃R₄Y< geschützten Weinsäureestern mit Li-organischen Verbindungen oder Grignardreagenzien, z.B. der Formel R₅Li oder R₅MgX mit X gleich Cl, Br oder I, herstellbar. Die Herstellung ist von D. Seebach et al. in Helv. Chim. Acta 70, Seite 954 (1987) beschrieben.

Die Umsetzung kann bei Temperaturen von -78 bis +100°C, bevorzugt -20 bis 40°C, vorzugsweise in einem inerten Lösungsmittel, wie z.B. Kohlenwasserstoffen (Toluol, Xylol), Ethern (Diethylether, Tetrahydrofuran, Dioxan, Ethylenglykoldimethylether) erfolgen. Zweckmässig wird die Reaktion unter Schutzgasatmosphäre, zum Beispiel Argon, und in Gegenwart einer basischen Verbindung durchgeführt, um das entstehende HZ zu binden. Geeignete basische Verbindungen sind zum Beispiel Alkalicarbonate, wie Natriumcarbonat und Natriumbicarbonat sowie Amine, besonders tertiäre Amine, wie z.B. Triethylamin. Weitere geeignete Basen sind LiH, KH, NaH, Lithiumsekundäramide (Lithiumdiisopropylamid), Lithiummethyl, Lithiumethyl, Lithium-, Natrium- und Kaliumhexamethyldisilazid, Methylmagnesiumchlorid oder Diethylzink. Die Reaktionslösung kann direkt weiterverwendet werden. Zur Isolierung der Verbindungen wird das ausgefallene Salz abfiltriert und das Lösungsmittel entfernt. Die so erhaltenen Rohprodukte können ohne Reinigung weiterverwendet werden.

Die Herstellung der Verbindungen der Formel I erfolgt zweckmässig bei Temperaturen von -78 bis +40°C, bevorzugt -20 bis 40°C, und in einem inerten Lösungsmittel. Geeignete Lösungsmittel sind insbesondere Ether. Die Reaktion wird unter Schutzgas, z.B. Argon durchgeführt. Ausgefallene Salze können abfiltriert werden. Das gegebenenfalls nach Entfernen des Lösungsmittels erhaltene Rohprodukt kann direkt weiterverwendet werden.

Die erfindungsgemässen Verbindungen eignen sich hervorragend als enantioselektive Reaktanden für Reaktionen mit prochiralen Aldehyd-, Keto- und/oder N-substituierten Imingruppen. Besonders vorteilhaft ist, dass je nach Wahl der R,R- bzw. S,S-Konfiguration an den C-Atomen C(1) und C(2) in Formel I beide Enantiomere hergestellt werden können. Sie eignen sich insbesondere zur Uebertragung von Allylgruppen, sowie Enol-, Enamin- und Enhydrazingruppen durch Aldolreaktion. Ein weiterer Gegenstand der Erfindung ist diese Verwendung. Bei den Carbonylverbindungen handelt es vorzugsweise um prochirale Aldehyde und Ketone. Als Reaktionsprodukte erhält man in hohen Ausbeuten chirale sekundäre oder tertiäre Alkohole bzw. sekundäre Amine oder Aminosäuren mit hohem Ueberschuss eines Enantiomeren. Die Synthese von chiralen Wirkstoffen im Bereich der Pharmaka und Agrarchemikalien hat grosse Bedeutung erlangt. Die erfindungsgemässen Verbindungen eignen sich zur Herstellung von entsprechenden Zwischenprodukten für die Synthese solcher Wirkstoffe oder um in der Endstufe der Synthese solcher Wirkstoffe Gruppen mit chiralen C-Atomen einzuführen. So können z.B. in hohen Ausbeuten Pheromone für die Insektenbekämpfung hergestellt werden. Als Beispiel sei (+)- oder (-)-Ipsenol genannt, das gemäss dem in der EP-A-0 254 685 beschriebenen Verfahren hergestellt werden kann unter Verwendung eines erfindungsgemässen Titankomplexes (siehe Beispiel 17).

Die erfindungsgemässen Verbindungen sind darüber hinaus preisgünstig aus billigen Ausgangsstoffen herstellbar. Als Derivate von natürlichen Verbindungen können sie mittels biologischer Abbaumethoden umweltfreundlich entsorgt werden, zumal Titan bzw. Titanoxid bekannterweise physiologisch unbedenklich ist.

Ein weiterer Gegenstand vorliegender Erfindung ist ein Verfahren zur Herstellung von bevorzugt chiralen sekundären und tertiären Alkoholen und sekundären Aminen, das dadurch gekennzeichnet ist, dass man vorzugsweise prochirale Aldehyde, Ketone, N-substituierte Aldimine oder Ketimine mit 1 Mol einer Verbindung der Formel I pro Mol Aldehyd-, Keto- oder Imingruppe umsetzt. Bei dem Substituenten im Imin kann es sich um die zuvor erwähnten Reste wie Alkyl, Alkenyl, Cycloalkyl, Aryl und Aralkyl handeln. Sind diese Reste durch Carboxyl oder Carboxylestergruppen substituiert, erhält man als Reaktionsprodukte Aminosäuren bzw. deren Ester. Weitere geeignete Substituenten sind z.B. R₆SO₂- und Acyl mit bevorzugt 1 bis 8 C-Atomen, das mit -F substituiert sein kann. Beispiele sind Acetyl, Mono-, Di- und Trifluoracetyl, Benzoyl und Fluorbenzoyl.

Die Reaktion wird vorteilhaft bei Temperaturen von -80 bis 30°C in Gegenwart eines inerten Lösungsmittels und unter Schutzgas durchgeführt. Zur Isolierung des Reaktionsproduktes wird zweckmässig hydrolysiert, das Produkt extrahiert und in üblicher Weise gereinigt. Geeignete inerte Lösungsmittel sind beispielsweise Ether oder Kohlenwasserstoffe, wie Pentan, Hexan, Cyclohexan, Methylcyclohexan, Benzol, Toluol, Xylol, Diethylether, Tetrahydrofuran, Dioxan, Nitrile, wie z.B. Acetonitril, oder chlorierte Kohlenwasserstoffe, wie z.B. Methylenchlorid, Chloroform oder Chlorbenzol.

Die nachfolgenden Beispiele erläutern die Erfindung. Cp steht für Cyclopentadienyl.

### A) Herstellungsbeispiele

### Beispiel 1:

### a) [(4S,5S)-2-(t-Butyl)-1,3-dioxolan-4,5-bis(diphenylmethoxy)]-cyclopentadienyl-chloro-titan.

Zu einer Lösung von 0,55 g (2,5 mmol) Cyclopentadienyl-titantrichlorid in 50 ml Diethylether werden 1,24 g (2,5 mmol) (4S,5S)-2-(t-Butyl)-4,5-[bis(diphenylhydroxymethyl)]-1,3-dioxolan zugegeben. Zu dieser Reaktionslösung wird innert 30 Minuten bei Raumtemperatur (RT) eine Lösung von 0,56 g (5,5 mmol) Triethylamin in 10 ml Diethylether zugetropft. Die hellgelbe Suspension wird bei RT für weitere 12 Stunden gerührt und das gebildete Triethylaminhydrochlorid unter Argon abgenutscht und dreimal mit 10 ml Ether gewaschen. Die so erhaltene Reaktionslösung kann direkt weiterverwendet werden. Zur Isolation des Komplexes wird das Lösungsmittel am Vakuum abdestilliert. Es werden zwei Diastereomere im Verhältnis 4:1 gebildet.

### Hauptisomeres.

¹H-NMR-Spektrum (CD₂Cl₂, 400 MHz): 7,20-7,70 (m, 20H, Ph); 6,26 (s, 5H, Cp); 4,86 (d, J = 6,5 1H, H-C(4) oder H-C(5)); 4,79 (d, J = 6,5, 1H, H-(C5) oder H-C(4)); 3,43 (s, 1H, H-C(2)); 0,84 (s, 9H, C(CH₃)₃).
¹³C-NMR-Spektrum (CD₂Cl₂, 100 MHz): 147,22 (s, Ph); 146,79 (s, Ph); 142,83 (s, Ph); 142,17 (s, Ph); 128,73 (d, Ph); 128,59 (d, Ph); 128,24 (d, Ph); 128,08 (d, Ph); 172,99 (d, Ph); 127,92 (d, Ph); 127,91 (d, Ph); 127,89 (d, Ph); 172,59 (d, Ph); 127,53 (d, Ph); 127,47 (d, Ph); 127,33 (d, Ph); 117,32 (d, Cp); 109,42 (d, C(2); 97,88 (s, C(Ph)₂O); 97,27 (s, C(Ph)₂O); 83,83 (d, C(4) oder C(5)); 80,30 (d, C(5) oder C(4)); 35,51 (s, C(CH₃)₃); 24,74 (q, C(CH₃)₃.

### Nebenisomeres.

¹H-NMR-Spektrum (CD₂Cl₂, 400 MHz); 7,20-7,70 (m, 20H, Ph); 6,56 (s, 5H, Cp); 5,03 (d, J = 7,5, 1H, H-C(4) oder H-C(5)); 4,78 (d, J = 7,5, 1H, H-(C5) oder H-C(4); 3,44 (s, 1H, H-C(2)); 0,61 (s, 9H, C(CH₃)₃).
¹³C-NMR-Spektrum (CD₂Cl₂, 100 MHz): 146,49 (s, Ph); 146,45 (s, Ph); 142,53 (s, Ph); 142,06 (s, Ph); 129,25 (d, Ph); 128,84 (d, Ph); 128,63 (d, Ph); 128,53 (d, Ph); 128,16 (d, Ph); 128,07 (d, Ph); 127,99 (d, Ph); 127,92 (d, Ph); 127,88 (d, Ph); 127,55 (d, Ph); 127,46 (d, Ph); 127,17 (d, Ph); 117,80 (d, Cp); 109,85 (d, C(2); 97,52 (s, C(Ph)₂O); 97,40 (s, C(Ph)₂O); 80,83 (d, C(4) oder C(5)); 80,01 (d, C(5) oder C(4)); 35,30 (s, C(CH₃)₃); 24,38 (q, C(CH₃)₃.

### b) [(4S,5S)-2-(t-Butyl)-1,3-dioxolan-4,5-bis(diphenylmethoxy)]-cyclopentadienyl-allyl-titan.

Die nach a) erhaltene Reaktionslösung wird auf 0°C abgekühlt und 1,8 ml (2,25 mmol) Allylmagnesiumchlorid (1,25 molare Lösung in Tetrahydrofuran) werden innert 5 Minuten zugetropft. Die gelb-braune Suspension wird 1 Stunde bei 0°C gerührt. Die so erhaltene Reaktionslösung wird direkt weiterverwendet.

### Beispiel 2:

### a) [(4R,5R)-2,2-Dimethyl-1,3-dioxolan-4,5-bis(diphenylmethoxy)]-cyclopentadienyl-chloro-titan.

Zu einer Lösung von 0,55 g (2,5 mmol) Cyclopentadienyl-titantrichlorid in 50 ml Diethylether werden 1,17 g (2,5 mmol) (4R,5R)-2,2-Dimethy-4,5-[bis-(diphenylhydroxymethyl)]-1,3-dioxolan zugegeben. Zu dieser Reaktionslösung wird innert 30 Minuten bei Raumtemperatur (RT) eine Lösung von 0,56 g (5,5 mmol) Triethylamin in 10 ml Diethylether zugetropft. Die hellgelbe Suspension wird bei RT für weitere 12 Stunden gerührt und das gebildete Triethylaminhydrochlorid unter Argon abgenutscht und dreimal mit 10 ml Ether gewaschen. Die so erhaltene Reaktionslösung wird direkt weiterverwendet.
¹H-NMR-Spektrum (CD₂Cl₂, 400 MHz): 7,65-7,27 (m, 20H, Ph); 6,43 (s, 5H, Cp); 5,16 (d, J=7,0, 1H, H-C(4) oder H-C(5)); 4,95 (d, J=7,0, 1H, H-C(5) oder H-C(4); 0,94 (s, 3H, CH₃); 0,50 (s, 3H, CH₃).
¹³C-NMR-Spektrum (CD₂Cl₂, 100 MHz): 148,87 (s, Ph); 146,72 (s, Ph); 142,86 (s, Ph); 142,72 (s, Ph); 129,75 (d, Ph); 129,08 (d, Ph); 128,60 (d, Ph); 128,36 (d, Ph); 127,99 (d, Ph); 127,92 (d, Ph); 127,73 (d, Ph); 127,68 (d, Ph); 127,59 (d, Ph); 127,57 (d, Ph); 127,55 (d, Ph); 127,49 d, Ph); 117,48 (d, Cp); 111,71 (s, C(2); 98,65 (s, C(Ph)₂O); 98,06 (s, C(Ph)₂O); 82,12 (d, C(4) oder C(5)); 81,67 (d, C(5) oder C(4)); 27,60 (q, (CH₃); 27,00 (q, (CH₃).

### b) [(4R,5R)-2,2-Dimethyl-1,3-dioxolan-4,5-bis(diphenylmethoxy)]-cyclopentadienyl-allyl-titan.

Die nach a) erhaltene Reaktionslösung wird auf 2°C abgekühlt und 1,8 ml werden (2,25 mmol) Allylmagnesiumchlorid (1,25 molare Lösung in Tetrahydrofuran) innert 5 Minuten zugetropft. Die orange Suspension wird 1 Stunde bei 2°C gerührt und unter Argon abgenutscht. Die so erhaltene Reaktionslösung wird direkt weiterverwendet.
¹H-NMR-Spektrum (CD₂Cl₂, 250MHz): 7,65-7,27 (m, 20H, Ph); 6,15 (s, 5H, Cp); 5,85 (quint. J=11,5, H-C(2')) 5,40 (d, J=7,0, 1H, H-C(4) oder H-C(5)); 4,78 (d, J=7,0, 1H, H-(C5) oder H-C(4); 3,36 (d, J= 11,5, 4H, H-C(1'), H-C(3')); 0,72 (s, 3H, CH₃); 0,58 (s, 3H, CH₃).
¹³C-NMR-Spektrum (CD₂Cl₂, 62,9 MHz): 148,48 (s,Ph); 147,86 (s,Ph); 143,85 (s,Ph); 143,35 (d, C(2')); 143,23 (s,Ph); 129,75 (d,Ph); 129,08 (d,Ph); 128,60 (d,Ph); 128,36 (d,Ph); 127,99 (d,Ph); 127,92 (d,Ph); 127,73 (d,Ph); 127,68 (d,Ph); 127,59 (d,Ph); 127,57 (d,Ph); 127,55 (d,Ph); 127,49 (d, Ph); 115,25 (d,Cp); 112,10 (s, C(2)); 94,95 (s, C(Ph)₂O); 88,03 (t, C(1'),C(3')); 82.30 (d, C(4) oder C(5)); 82.25 (d, C(5) oder C(4)); 27.67 (q, (CH₃); 27.64 (q, (CH₃).

### Beispiel 3

### a) [(4S,5S)-2,2-Dimethyl-1,3-dioxolan-4,5-bis(diphenylmethoxy)]-cyclopentadienyl-chloro-titan

wird ausgehend von (4S,5S)-2,2-Dimethyl-4,5-[bis(diphenylhydroxymethyl)]-dioxolan gemäss Beispiel 2a hergestellt.

### b) [(4S,5S)-2,2-Dimethyl-1,3-dioxolan-4,5-bis(diphenylmethoxy)]-cyclopentadienyl-allyl-titan

wird ausgehend von [(4S,5S)-2,2-Dimethyl-1,3-dioxolan-4,5-bis(diphenyl-methoxy)]-cyclopentadienylchloro-titan gemäss Beispiel 2b hergestellt.

### Beispiel 4

### a)[(4R,5R)-2,2-Dimethyl-1,3-dioxolan-4,5-bis(diphenylmethoxy)]cyclopentadienyl-E-crotyl-titan.

Zu 10mmol [(4R,5R)-2,2-Dimethyl-1,3-dioxolan-4,5-bis(diphenylmethoxy)]cyclopentadienyl-chloro-titan, hergestellt analog Beispiel 2a, in 160ml Diethylether wird bei -10°C 10ml (10mmol) (Crotyl)MgCl (1 molare Lösung in Diethylether) zugegeben. Die rostrote Suspension wird 2h bei dieser Temperatur gerührt und unter Argon abgenutscht. Die so erhaltene Reaktionslösung kann eingeengt oder direkt weiterverwendet werden.
¹H-NMR-Spektrum (CD₂Cl₂, 250MHz): 7,65-7.15 (m, 20H, Ph); 6,08 (s, 5H, Cp); 5,46 (dxtxq, J=14,0, 8,5, 1,5, H-C(2')); 4,82 (dxq, J= 14,0, 6,5, H-C(3')); 5,11 (d, J=7,0, 1H, H-C(4) oder H-C(5)); 4,47 (d, J=7,0, 1H, H-(C5) oder H-C(4); 2,26 (dxd, J=8,5, 8,5 H-C(1')); 2,17 (dxd, J=8,5, 8,5 H-C(1')); 1,67 (dxd, 3H, J=6,5, 1,5, H-(C4')); 0,68 (s, 3H, C(2)-CH₃); 0,58 (s, 3H, C(2)-CH₃).

### Beispiel 5:

### a) (4R,5R)-2,2-Dimethyl-4,5-[bis-(di-pentafluorphenylhydroxymethyl)]-1,3-dioxolan.

Zu einer Lösung von Brompentafluorbenzol 1,25ml (10mmol) in 30ml Diethylether wird bei -78°C 6,25ml (10mmol) n-ButylLi in Hexan innert 45min zugetropft, auf Raumtemperatur (RT) erwärmt, gerührt und auf 0°C abgekühlt. Zu dieser Reaktionslösung wird 10mmol (4R,5R)-2,2-Dimethyl-1,3-dioxolan-4,5-dicarbonsäure-diethylester, gelöst in 20ml Diethylether langsam zugetropft, 12h bei RT gerührt und durch langsame Zugabe einer gesättigten NH₄Cl Lösung hydrolisiert. Die Emulsion wird 3x mit 80ml Diethylether extrahiert, mit einer gesättigten NaCl Lösung gewaschen über Na₂SO₄ getrocknet und eingedampft. Das Produkt wird durch Flash-Chromatographie (80g SiO₂, Ether/Hexan 1:5) gereinigt.
Ausbeute: 4,63g (56%).
¹H-NMR-Spektrum (CDCl₃, 250 MHz): 5,43 (s, 2H); 4,95 (s, 2H); 1,40 (s, 6H).

### b) [(4R,5R)-2,2-Dimethyl-1,3-dioxolan-4,5-bis(di-pentafluorphenylmethoxy)]-cyclopentadienyl-chloro-titan.

Wird analog Beispiel 2a, jedoch ausgehend von (4R,5R)-2,2-Dimethyl-4,5-[bis-(di-pentafluorphenylhydroxymethyl)]-1,3-dioxolan hergestellt.

### c) [(4R,5R)-2,2-Dimethyl-1,3-dioxolan-4,5-bis(di-pentafluorphenylmethoxy)]-cyclopentadienyl-allyl-titan.

Die nach Beispiel 5b erhaltene Reaktionslösung wird analog Beispiel 2b umgesetzt und kann direkt weiter verwendet werden.

### Beispiel 6:

### a) (4R,5R)-2,2-Dimethyl-4,5-[bis-(di-2-furylhydroxymethyl)]-1,3-dioxolan.

Eine Lösung von 97ml n-ButylLi (155mmol) in Hexan wird mit 100ml Diethylether verdünnt, auf -20°C abgekühlt und mit 11,62ml (160mmol) Furan innerhalb von 10min versetzt. Die Reaktionslösung wird langsam auf RT erwärmt, und anschliessend während 4h am Rückfluss erwärmt. Zu dieser Reaktionslösung wird 9,84g (40mmol) (4R,5R)-2,2-Dimethyl-1,3-dioxolan-4,5-dicarbonsäure-diethylester, gelöst in 20ml Ether, unter Eiskühlung langsam zugetropft, anschliessend 2h bei RT und 16h unter Rückfluss gerührt und durch langsame Zugabe von 300ml einer gesättigten NH₄Cl Lösung hydrolisiert. Die Emulsion wird über Hyflo filtriert, 3x mit 200ml Diethylether extrahiert, mit einer gesättigten NaCl Lösung gewaschen, über Na₂SO₄ getrocknet und eingedampft. Das Produkt wird durch Flash-Chromatographie (800g SiO₂, Toluol/Ether 10:1) gereinigt. Ausbeute: 5,10g (30%).
¹H-NMR-Spektrum (CDCl₃, 250 MHz): 7,48-7,36 (m, 4H); 6,48-6,30 (m, 8H); 4,78 (s, 2H); 1,02 (s, 6H).
¹³C-NMR-Spektrum (CDCl₃, 62,9 MHz): 154,3(s), 152,9 (s), 142,4 (d); 111,5 (s); 110,6 (d); 110,3 (d); 109,8 (d), 109,3(d); 80,4 (d); 72,6 (s); 26,7 (q).

### b) [(4R,5R)-2,2-Dimethyl-1,3-dioxolan-4,5-bis(di-2-furylmethoxy)]-cyclopentadienyl-chloro-titan.

Wird analog Beispiel 2a jedoch ausgehend von (4R,5R)-2,2-Dimethyl-4,5-[bis-(di-2-furylhydroxymethyl)]-1,3-dioxolan hergestellt.

### c) [(4R,5R)-2,2-Dimethyl-1,3-dioxolan-4,5-bis(di-2-furylmethoxy)]-cyclopentadienyl-allyl-titan.

Die nach Beispiel 6b erhaltene Reaktionslösung wird analog Beispiel 2b umgesetzt und kann direkt weiter verwendet werden.

### Beispiel 7:

### a) (4R,5R)-2,2-Dimethyl-4,5-[bis-(di-2-propenylhydroxymethyl)]-1,3-dioxolan

13,8g (575mmol) Mg-Späne werden in 50 ml Tetrahydrofuran (THF) vorgelegt und 43,7ml 500mmol 2-Brompropen innerhalb von 1,5h zugetropft und 1h am Rückfluss erwärmt und auf 40°C abgekühlt. Zur Reaktionslösung wird 24,6g (100mmol) (4R,5R)-2,2-Dimethyl-1,3-dioxolan-4,5-dicarbonsäure-diethylester, gelöst in 50 ml THF innerhalb 1h zugetropft. Anschliessend wird mit 250ml 2n H₂SO₄ hydrolisiert, die Phasen getrennt und die Wasserphase 3x mit 200ml Diethylether extrahiert, die vereinigten Etherphasen werden 2 x mit einer gesättigten NaCl Lösung gewaschen, über NaSO₄ getrocknet und eingedampft. Das Produkt wird durch Flash-Chromatographie (1kg SiO₂, Ether/Hexan 1:5) gereinigt. Ausbeute: 6,1g (18.9%).
¹H-NMR-Spektrum (CDCl₃, 250 MHz): 5,25 (dxq, J=1,5, 0,5, 2H); 5,19 (dxq, J= 1,5, 0,5, 2H); 5,11 (dxq, J= 1,5, 1,5, 2H); 5,00 (dxq, J=1,5, 1,5, 2H); 4,40 (s, 2H); 1,80 (dxd J= 1,5, 0,5, 6H) 1,72 (dxd J= 1,5, 0,5, 6H); 1,40 (s, 6H)
¹³C-NMR-Spektrum (CDCl₃, 62,9 MHz): 146,1(s); 145,7 (s); 114,3 (t); 113,7(t); 98,4 (s); 80,2 (d); 79,1 (s); 27,2 (q); 21,0(q); 19,4 (q).

### b) [(4R,5R)-2,2-Dimethyl-1,3-dioxolan-4,5-bis-(di-2-propenyl-methoxy)]-cyclopentadienyl-chloro-titan.

Wird ausgehend von (4R,5R)-2,2-Dimethyl-4,5-[bis-(di-2-propenylhydroxymethyl)]-1,3-dioxolan analog Beispiel 2a hergestellt.

### c) [(4R,5R)-2,2-Dimethyl-1,3-dioxolan-4,5-bis-(di-2-propenyl-methoxy)]-cyclopentadienyl-allyl-titan.

Die nach Beispiel 7b erhaltene Reaktionslösung wird analog Beispiel 2b umgesetzt und kann direkt weiter verwendet werden.

### Beispiel 8

### [(4R,5R)-2,2-Diphenyl-1,3-dioxolan-4,5-bis(diphenylmethoxy)]-cyclopentadienyl-chloro-titan

Wird ausgehend von (4R,5R)-2,2-Diphenyl-4,5-[bis-(diphenylhydroxymethyl)]-1,3-dioxolan analog Beispiel 2a hergestellt.
¹H-NMR-Spektrum (CDCl₃, 250MHz): 7,70-6,40 (m, 30H, Ph); 6,29 (s, 5H, Cp); 5,33 (d, J=7,0, 1H, H-C(4) oder H-C(5)); 5,20 (d, J=7,0, 1H, H-(C5) oder H-C(4);
¹³C-NMR-Spektrum (CDCl₃, 62,89MHz): 146,4 (s,Ph); 146,3 (s,Ph); 144,0 (s,Ph); 143,4 (s,Ph); 141,3 (s,Ph); 140,2 (s,Ph); 128,6-126,3 (d, 16C, Ph); 125,4 (d, Ph); 125,2 (d, Ph); 116,8 (d, Cp); 112,2 (s, C(2); 98,4 (s, C(Ph)₂O); 97,8 (s, C(Ph)₂O); 84,3 (d, C(4) oder C(5)); 83,5 (d, C(5) oder C(4)).

### Beispiel 9

### a) [(4R,5R)-2,2-Dimethyl-1,3-dioxolan-4,5-bis(diphenylmethoxy)]-pentamethylcyclopentadienyl-chloro-zirkon

Zu einer Lösung von 6,66g (20mmol) Pentamethylcyclopentadienyl-zirkontrichlorid in 150ml Diethylether werden 9,32g (20mmol) (4R,5R)-2,2-Dimethyl-4,5-[bis(diphenylhydroxymethyl)]-1,3-dioxolan zugegeben. Zu dieser Reaktionslösung wird innert 30 Minuten bei Raumtemperatur (RT) eine Lösung von 6,1ml (44 mmol) Triethylamin in 50ml Diethylether zugetropft. Die beige Suspension wird bei RT für weitere 12h gerührt und das gebildete Triethylaminhydrochlorid unter Argon abgenutscht und dreimal mit ca 20ml Diethylether gewaschen. Die so erhaltene Reaktionslösung kann direkt weiterverwendet werden.
¹H-NMR-Spektrum (CDCl₃, 250MHz): 7,55-7,18 (m, 20H, Ph); 5,12 (d, J=7,0, 1H, H-C(4) oder H-C(5)); 4,92 (d, J=7,0, 1H, H-(C5) oder H-C(4); 1,95 (s, 15H, Cp*); 0,46 (s, 3H, CH₃); 0,45 (s, 3H, CH₃).
¹³C-NMR-Spektrum (CDCl₃, 62,89MHz): 148,5 (s,Ph); 148,2 (s,Ph); 143,6 (s,Ph); 143,4 (s,Ph); 129,8 (d,Ph); 129,5 (d,Ph); 128,6-127,5 (d, 10C, Ph); 123,74 (s, Cp*); 111,62 (s, C(2); 91,38 (s, C(Ph)₂O); 90,19 (s, C(Ph)₂O); 82,16 (d, C(4) oder C(5)); 80,55 (d, C(5) oder C(4)); 27,50 (q, (CH₃); 27,40 (q, (CH₃); 11,10 (q, Cp*).
Ph: Phenyl
Cp*: Cyclopentadienyl

### b) [(4R,5R)-2,2-Dimethyl-1,3-dioxolan-4,5-bis(diphenylmethoxy)]pentamethylcyclopentadienyl-methyl-zirkon

Eine Lösung von 5mmol der Zirkonverbindung von Beispiel 9a in 60 ml Diethylether wird auf -74°C abgekühlt, mit 1,5 ml einer 3 molaren CH₃MgBr Lösung in THF versetzt und 2h bei -74°C gerührt. Die so erhaltene Reaktionslösung wird direkt weiter verwendet.

### Beispiel 10

### a) [(4R,5R)-2,2-Dimethyl-1,3-dioxolan-4,5-bis(diphenylmethoxy)]pentamethylcyclopentadienyl-chloro-hafnium

Wird analog Beispiel 9a) jedoch ausgehend von Pentamethylcyclopentadienyl-hafnium-trichlorid hergestellt.
¹H-NMR-Spektrum (CDCl₃, 250MHz): 7,55-7.2 (m, 20H, Ph); 5,08 (d, J=7,0, 1H, H-C(4) oder H-C(5)); 4,92 (d, J=7,0, 1H, H-(C5) oder H-C(4); 2.00 (s, 15H, Cp*); 0,46 (s, 3H, CH₃); 0,43 (s, 3H, CH₃).
¹³C-NMR-Spektrum (CDCl₃, 62,89MHz): 148,63(s,Ph); 148,18 (s,Ph); 143,84 (s,Ph); 143,70 (s,Ph); 129,8 (d,Ph); 129,1 (d,Ph); 128,6-127,5 (d, 10C, Ph); 122,14 (s, Cp*); 112,01 (s, C(2); 90,34 (s, C(Ph)₂O); 90,19 (s, C(Ph)₂O); 82,17 (d, C(4) oder C(5)); 80,58 (d, C(5) oder C(4)); 27,56 (q, (CH₃); 27,40 (q, (CH₃); 10,87 (q, Cp*).

### b) [(4R,5R)-2,2-Dimethyl-1,3-dioxolan-4,5-bis(diphenylmethoxy)]pentamethylcyclopentadienyl-methyl-hafnium

Wird analog Beispiel 9b hergestellt jedoch ausgehend von der Hafniumverbindug von Beispiel 10a.

### B) Anwendungsbeispiele:

### Beispiel 11: Herstellung von (S)-1-Phenyl-3-buten-1-ol

Eine Lösung von 1,25 mmol der Titanverbindung von Beispiel 2b) in 60 ml Diethylether wird auf -74°C abgekühlt und mit 0,1 ml (1,0 mmol) Benzaldehyd (frisch destilliert) versetzt. Die schwach gelbe, klare Lösung wird 2 Stunden bei -74°C weitergerührt und mit 5 ml einer 5N H₂O/THF Lösung versetzt, auf RT erwärmt, eine weitere Stunde bei dieser Temperatur gerührt, genutscht und am Rotavapor eingeengt. Das Rohprodukt (0,80 g Klares farbloses Oel) wird flash-chromatographiert (60 g Kieselgel, 35 cm lang, ⌀ 2cm, 5 ml/Fraktion, 0,4 bar, Toluol/Ethanol 10:1). Man erhält 0,098 g Produkt. Ausbeute: 66 %, ee: 93 %.

### Beispiel 12: Herstellung von (S)-1-Tridecen-4-ol

Eine Lösung von 1,25 mmol der Titanverbindung von Beispiel 2b) in 60 ml Diethylether wird auf -74°C abgekühlt und mit 0,19 ml (0,1 mmol) Caprinaldehyd (frisch destilliert) versetzt. Die schwach gelbe, klare Lösung wird 2 Stunden bei -74°C weitergerührt und mit 5 ml einer 5N H₂O/THF Lösung versetzt, auf RT erwärmt, eine weitere Stunde bei dieser Temperatur gerührt, genutscht und am Rotavapor eingeengt. Das Rohprodukt (1,13 g klares farbloses Oel) wird flash-chromatographiert (60 g Kieselgel, 35 cm lang ⌀ 2 cm, 5 ml/Fraktion, 0,4 bar, Toluol/Ethanol 10:1) und anschliessend am Kugelrohr destilliert (Kp: ≈ 120-130 bei 0,104 mbar). Man erhält 0,141 g Produkt. Ausbeute: 71 %, ee: 95 %.

### Beispiel 13: Herstellung von (R)-1-Phenyl-3-buten-1-ol

Eine Lösung von 2,25 mmol Titanverbindung gemäss Beispiel 1b) in 120 ml Diethylether wird auf -74°C abgekühlt und mit 0,2 ml (2,0 mmol) Benzaldehyd (frisch destilliert) versetzt. Die beige Suspension wird 2 Stunden bei -74°C weitergerührt und mit 5 ml einer 5N H₂O/THF Lösung versetzt. Die beige Suspension wird auf RT erwärmt, eine weitere Stunde bei dieser Temperatur gerührt, genutscht und am Rotavapor eingeengt. Das Rohprodukt (1,53 g gelbbraunes Oel) wird zweimal flash-chromatographiert (80 g Kieselgel, 25 cm lang ⌀ 2cm, 10 ml/Fraktion, 0,5 bar, Toluol/Ethanol 20:1); (50 g Kieselgel, 25 cm lang, ⌀ 2 cm, 15 ml/Fraktion, 0,5 bar, Toluol/Ethanol 15:1). Man erhält 0,2 g Produkt. Ausbeute: 66 %, ee: 82 %.

### Beispiel 14: Herstellung von (2S,3R)-2-(tert.Butoxycarbonylamino)-3-hydroxy-hexansäure-ethylester

Zu einer Lösung von 1,9 g Cyclohexylisopropylamin in 60 ml Tetrahydrofuran werden bei -20 bis -30°C 7,6 ml einer 1,6 M Lösung von Butyl-Lithium in Hexan gegeben. Nach 20 min. kühlt man auf -78°C und tropft eine Lösung von 3,0 g 1,1,3,3-Tetramethyl-1,3-disila-azolidin-N-essigsäure-ethylester in 60 ml Tetrahydrofuran zu. Nach 1 Stunde Rühren bei -78°C werden 161 ml einer 0,083 M Lösung von [(4R,5R)-2,2-Dimethyl-1,3-dioxolan-4,5-bis(diphenylmethoxy)]cyclopentadienyl-chloro-titanat (gemäss Beispiel 2a) in Ether zugetropft. Man rührt während 43 Stunden bei -78°C und gibt die Lösung anschliessend via Stahlkanüle zu einer auf -78°C gekühlten Lösung von 0,97 g Butyraldehyd in 15 ml Tetrahydrofuran. Nach 22 Stunden Rühren bei -78°C werden 1,5 ml Wasser zugegeben, auf Raumtemperatur aufgewärmt und filtriert. Das Filtrat versetzt man mit 24 ml Wasser und 4,8 ml Essigsäure, rührt 2 Stunden bei Raumtemperatur und dampft im Vakuum ein. Eine Probe (5 mg) des Rückstandes wird mit Trifluoressigsäureanhydrid in Methylenchlorid während 2 Stunden deriva- tisiert und durch Kapillar-Gaschromatographie (Chirasil-Val, 90-180°C/2°C pro min.) analysiert. Die Probe besteht demgemäss aus 90,8 % (2S,3R)-Isomer (Retentionszeit 11,7 min.) und 9,2 % (2R,3S)-Isomer (Retentionszeit 10,8 min.). Der Rest des Rohproduktes wird mit 250 ml Ether versetzt und dreimal mit je 100 ml 0,1 N Salzsäure geschüttelt. Die Wasserphase wird zweimal mit je 250 ml Ether extrahiert mit Natronlauge auf pH 4 eingestellt und im Vakuum bei Raumtemperatur auf 50 ml eingeengt. Nach Zugabe von 80 ml Dioxan, 6,7 g Natriumbicarbonat und 5,3 g di-tert.Butyldicarbonat rührt man 15 Stunden bei Raumtemperatur. Das Reaktionsgemisch wird mit 250 ml Diethylether verdünnt, zweimal mit je 250 ml Wasser und gesättigter Kochsalzlösung gewaschen, und die Wasserphasen werden zweimal mit je 250 ml Ether extrahiert. Die organischen Extrakte werden getrocknet (Na₂SO₄) und eingedampft. Die Chromatographie des Rückstandes (Kieselgel, Essigester/Hexan 1:4 bis 1:2) gibt 2,09 g (2S,3R)-tert.Butoxycarbonylamino-3-hydroxy-hexansäure-ethylester.

### Beispiel 15: Herstellung von (4R,1'S)-2,2-Dimethyl-1,3-dioxolan-4-(1'-hydroxy-3'-buten-1'-yl)

Eine Lösung von 4,5 mmol der Titanverbindung von Beispiel 2b in 70 ml Diethylether wird auf -74°C abgekühlt und mit 0,52 g (4,0 mmol) frisch hergestelltem 2,3-O-Isopropylidene-D-glyceraldehyd versetzt. Die orange-gelbe Suspension wird 4 Stunden bei -74°C weitergerührt und mit 30 ml einer wässrigen Ammoniumfluorid-Lösung (45 %) versetzt, auf Raumtemperatur erwärmt, über Nacht bei dieser Temperatur gerührt und über Hyflo genutscht. Die Emulsion wird mit 50 ml Diethylether verdünnt, anschliessend die organische Phase abgetrennt, gewaschen und über Na₂SO₄ getrocknet. Das Rohprodukt (2,5 g klares, gelbliches Oel) wird in 10 ml Pentan suspendiert, 1 Stunde bei Raumtemperatur gerührt und genutscht. Das Filtrat wird flash-chromatographiert (60 g Kieselgel, 20 cm lang, ⌀ 2 cm, 30 ml/Fraktion, 0,4 bar, Diethylether/Pentan 1:2) Ausbeute: 0,2 g ee>95 %.

### Beispiel 16: Herstellung von (4R,1'R)-2,2-Dimethyl-1,3-dioxolan-4-(1'-hydroxy-3'-buten-1'-yl)

Die Herstellung erfolgt gemäss Beispiel 15, aber unter Verwendung der Titanverbindung von Beispiel 3b. Ausbeute: 0,26 g ee >95 %.

### Beispiel 17: Herstellung von (R)-3-Hydroxy-5-methylhexansäure

Zu einer Lösung von 1,12 ml Diisopropylamin in 15 ml trockenem Diethylether (Ether) werden bei -25°C 4,4 ml 1,6 M Butyl-Lithium in Hexan gegeben (Argon). Nach 25 min. bei -20°C wird auf -78°C gekühlt und 805 »l Essigsäure-tert.butylester in 10 min. mit der Spritze zugetropft. Nach 45 min. Rühren bei -78°C wird eine frisch zubereitete auf -78°C vorgekühlte Lösung von 4,9 g [(4R,5R)-2,2-Dimethyl-1,3-dioxolan-4,5-bis-(diphenylmethoxy)]cyclopentadienyl-chloroti tanat (gemäss Beispiel 2a) in ca. 100 ml Ether via Kanüle in 15 min. zugegeben. Man rührt 30 min. bei -78°C und 3 Stunden bei -30°C, kühlt auf -78°C und gibt 650 »l Isovaleraldehyd in 5 min. mit der Spritze zu. Nach 2 Stunden bei -78°C werden 20 ml 45 % NH₄F-Lösung zugegeben und das Gemisch wird auf Raumtemperatur erwärmt. Nach 17 Stunden Rühren wird filtriert, das Filtrat wird mit 10 ml gesättigter Kochsalzlösung versetzt und dreimal mit je 100 ml Ether extrahiert. Die organischen Phasen werden mit gesättigter NaCl-Lösung und 1N HCl gewaschen, getrocknet (MgSO₄) und eingedampft. Der Rückstand (5,1 g) wird in 6 ml 2N Natronlauge und 12 ml Methanol 2 Stunden bei 50°C gerührt. Nach dem Abdampfen von Methanol im Vakuum wird dreimal mit je 50 ml Ether extrahiert. Die mit konzentrierter Salzsäure angesäuerte Wasserphase wird dreimal mit je 100 ml Essigester extrahiert. Die Essigesterphasen werden mit zweimal 50 ml gesättigter Kochsalzlösung gewaschen, getrocknet (MgSO₄) und eingedampft. Der Rückstand, 835 mg 1-(R)-3-Hydroxy-5-methyl-hexansäure mit 78 % optischer Reinheit, wird zweimal aus Cyclohexan (60 ml, 150 ml) umkristallisiert. Ausbeute: 595 mg Produkt mit 96 % optischer Reinheit, Smp. 86°C; [|[α]_{D}=-14,7° (c=1, CHCl₃). Gemäss der Vorschrift in der EP-A-0 254 685 (Beispiel 41d bis 41g) kann hieraus (+)-(R)-Ipsenol hergestellt werden.

### Beispiel 18: Herstellung von (S)-1-Phenyl-3-buten-1-ol

Analog Beispiel 11 jedoch ausgehend von:
[(4R,5R)-2,2-Dimethyl-1,3-dioxolan-4,5-bis-(di-pentafluorphenyl-methoxy)]-cyclopentadienyl-allyl-titan (hergestellt nach Bsp 5c). Ausbeute: 62%, (ee: 53.7%).

### Beispiel 19: Herstellung von (S)-1-Phenyl-3-buten-1-ol

Analog Beispiel 11 jedoch ausgehend von:
[(4R,5R)-2,2-Dimethyl-1,3-dioxolan-4,5-bis-(di-2-furylmethoxy)]-cyclopentadienyl-chloro-titan (hergestellt nach Bsp 6c). Ausbeute: 63%, (ee: 65.5%).

### Beispiel 20: Herstellung von (S)-1-Phenyl-3-buten-1-ol

Analog Beispiel 11 jedoch ausgehend von:
[(4R,5R)-2,2-Dimethyl-1,3-dioxolan-4,5-bis-(di-2-propenylmethoxy)]-cyclopentadienyl-chloro-titan (hergestellt nach Bsp 7c). Ausbeute: 53%, (ee: 71.3%).

### Beispiel 21: Herstellung von tert-Butyl (4S,1'S)-2,2-Dimethyl-4-(1'-hydroxy-3'-butenyl) oxazolidin-3-carboxylat

Eine Lösung 65 ml (4,5mmol) [(4R,5R)-2,2-Dimethyl-1,3-dioxolan-4,5-bis-(diphenyl-methoxy)]-cyclopentadienyl-allyl-titan (hergestellt nach Beispiel 2b) wird auf -74°C abgekühlt und 917 mg (4 mmol) tert-Butyl (4S)-2,2-Dimethyl-4-formyl-oxazolidin-3-carboxylat gelöst in 5ml Diethylether zugetropft. Die schwach gelbe, klare Lösung wird 3h bei -74°C weitergerührt und mit 25ml einer wässrigen 45%-igen NH₄F Lösung versetzt, auf RT erwärmt, weitere 12h bei dieser Temperatur gerührt (weisser Niederschlag) und über Celite filtriert. Die wässrige Phase wird abgetrennt und mit 100ml Diethylether extrahiert. Die die organische Phase wird 2x mit 50ml gesättigter NaCl-Lösung gewaschen, mit MgSO₄ getrocknet und eingedampft.
Das Produkt wird durch zweimalige Flash-Chromatographie (220g SiO₂, Hexan/Essigester 2.5:1) bezw. (20g SiO₂, Hexan/Essigester 2.5/:1) gereinigt.
Ausbeute: 1.007g, 93%, de (ee) 95%, [α]_{D}^{Rt}= -17.0° (c=0.95, C₆H₆).
¹³C-NMR (C₆D₆, 62,9 MHz, 340K) 24,1(CH₃); 27,0 (CH₃); 28,4 (C(CH₃)₃; 36,1 (C(2')); 61,9 (C(4)); 64,6 (C(5)); 72,3 (C( 1')); 80,4 (C(CH₃)₃; 94,3 (C(2)); 117,0 (C(4')); 135,7 (d, C(3'); 153,8 (COO-t-Bu).
¹H-NMR (C₆D₆, 250MHz, 340K): 1,39 (s, C(CH₃)₃), 1,45 (s, CH₃); 1,62 (s, CH₃); 2,10 (dxdxdxdxd, 1H, J= 14,0, 7,0, 7,0, 1,0, 1,0, H-C(2'); 2,30 (dxdxdxdxd, 1H, J= 14,0, 6,5, 3,0, 1,0, 1,0, H-C(2');3,59-3,73 (m, 2H, H-C(5)); 3,84-3,96 (m, H-C(4), H-C(1'); 4,97-5,08 (m, 2H, H-C(4')); 5,92 (dxdxdxd J=17,0, 10,0, 7,0, 6,5, H-C(3')).

### Beispiel 22: tert-Butyl (4S,1'R)-2,2-Dimethyl-4-(1'hydroxy-3'-butenyl)oxazolidin-3-carboxylat

Wird analog Beispiel 21 hergestellt jedoch unter Verwendung von [(4S,5S)-2,2-Dimethyl-1,3-dioxolan-4,5-bis-(diphenylmethoxy)]-cyclopentadienyl-allyl-titan (Beispiel 3b).
Ausbeute: 1.037g, 95%, de (ee) 99%, [α]_{D}^{Rt}=+4.4° (c=0.985, C₆H₆).
¹H-NMR (C₆D₆, 250MHz, 340K): 1,39 (s, C(CH₃)₃), 1,46 (s, CH₃); 1,62 (s, CH₃); 2,16-2,23 (m, 2H, H-C(2'); 3,63 (dxd, 1H, 9,0, 6,5, H-C(5)); 3,80-3,92 (m, 3H, H-C(1'), H-C(4), H-C(5)); 4,97-5,12 (m, 2H, H-C(4')); 5,90 (dxdxdxd J= 17,0, 10,0, 6,50, 6,5, H-C(3'));
¹³C-NMR(C₆D₆, 62,9 MHz, 340K) 24,2 (q,CH₃); 27,0 (q, CH₃); 28,4 (q, t-Bu); 38,9 (t, C(2')); 62,0 (d, C(4)); 64,5 (t, C(5)); 72,0 (d, C(1')); 80,1 (s,t-Bu); 94,4 (s, C(2)); 116,9 (t, C(4')); 135,9 (d, C(3'); 153,8(s)).

### Beispiel 23: Herstellung von (2S,3R)-2-(tert.Butoxycarbonylamino)-3-hydroxy-hexansäure-ethylester.

Zu einer Lösung von 1,9 g Cyclohexylisopropylamin in 60 ml Tetrahydrofuran werden bei -20 bis -30 °C 7,6 ml einer 1,6 M Lösung von Butyl-Lithium in Hexan gegeben. Nach 20 min. kühlt man auf -78 °C und tropft eine Lösung von 3,0 g 1,1,3,3-Tetramethyl-1,3-disila-azolidin-N-essigsäure-ethylester in 60 ml Tetrahydrofuran zu. Nach 1h Rühren bei -78°C werden 161 ml einer 0,083 M Lösung von [(4R,5R)-2,2-Dimethyl-1,3-dioxolan-4,5-bis(diphenylmethoxy)]cyclopentadienyl-chloro-titanat (gemäss Beispiel 2a) in Diethylether zugetropft. Man rührt während 43h bei -78°C und gibt die Lösung anschliessend über eine Stahlkanüle zu einer auf -78°C gekühlten Lösung von 0,97 g Butyraldehyd in 15 ml Tetrahydrofuran. Nach 22h Rühren bei - 78 °C werden 1,5 ml Wasser zugegeben, auf Raumtemperatur aufgewärmt und filtriert. Das Filtrat versetzt man mit 24 ml Wasser und 4,8 ml Essigsäure, rührt 2h bei Raumtemperatur und dampft schonend im Vakuum ein. Eine Probe (5 mg) des Rückstandes wird mit Trifluoressigsäureanhydrid in Methylenchlorid während 2h umgesetzt und durch Kapillar-Gaschromatographie (Chirasil-Val, 90 - 180°C/2°C pro min) analysiert. Die Probe besteht demgemäss aus 90,8% (2S,3R)-Isomer (Retentionszeit 11,7 min.) und 9,2% (2R,3S)-Isomer (Retentionszeit 10,8 min.). Der Rest des Rohproduktes wird mit 250 ml Diethylether versetzt und dreimal mit je 100 ml 0.1 N Salzsäure geschüttelt. Die Wasserphase wird zweimal mit je 250 ml Diethylether extrahiert, mit Natronlauge auf pH 4 eingestellt und im Vakuum bei Raumtemperatur auf etwa 50 ml eingeengt. Nach Zugabe von 80 ml Dioxan, 6,7 g Natriumbicarbonat und 5,3 g di-tert.Butyl-dicarbonat rührt man 15h bei Raumtemperatur. Das Reaktionsgemisch wird mit 250 ml Diethylether verdünnt, zweimal mit je 250 ml Wasser und gesättigter Kochsalzlösung gewaschen, und die Wasserphasen werden zweimal mit je 250 ml Diethylether extrahiert. Die organischen Extrakte werden getrocknet (Na₂SO₄) und eingedampft. Chromatographie des Rückstandes (Kieselgel, Essigester/Hexan 1:4 bis 1:2) gibt 2,09 g (2S,3R)-tert.Butoxycarbonylamino-3-hydroxy-hexansäure-ethylester.

### Beispiel 24: Herstellung von (2S,3R)-2-(tert.Butoxycarbonylamino)-3-hydroxy-hexansäure-tert.butylester

### a) 1,1,3,3-Tetramethyl-1,3-disila-azolidin-N-essigsäure-tert.butylester

Unter Stickstoffatmosphäre werden zu einer Aufschlämmung von 14,0 g Glycin-tert.butylester-hydrochlorid in 200 ml Dichlormethan unter Eiskühlung langsam 44,0 ml Triethylamin und eine Lösung von 23,4 g 1,2-Bis-(chlordimethylsilyl)-ethan in 100 ml Dichlormethan getropft. Nach 19 h Rühren bei Raumtemperatur werden weitere 7 ml Trietylamin und 5,4 g 1,2-Bis-(chlordimethylsilyl)-ethan zugesetzt und nochmals 22 h gerührt. Das Reaktionsgemisch wird mit Pufferlösung (0,41 M Na₂HPO₄ und 0,28 M KH₂PO₄ in H₂O), H₂O und gesättigten Kochsalzlösung gewaschen; die wässrigen Phasen werden zweimal mit Dichlormethan extrahiert. Man trocknet die organischen Phasen mit Na₂SO₄, dampft sie ein und destilliert den Rückstand: 12,7 g 1,1,3,3-Tetramethyl-1,3-disila-azolidin-N-essigsäure-tert.butylester (60-63°C; 0,02 mbar).

### b) (2S,3R)-2-(tert.Butoxycarbonylamino)-3-hydroxy-hexansäure-tert.butylester

Zu einer analog zu Beispiel 23 aus 0,93 g Cyclohexylisopropylamin, 30 ml THF und 3,8 ml Butyllithium (1,6 M in Hexan) hergestellten Lösung von Lithium-cyclohexylisopropylamid werden bei -78°C 1,5 g 1,1,3,3-Tetramethyl-1,3-disila-azolidin-N-essigsäure-tert.butylester in 30 ml THF getropft. Nach 1h Rühren bei -78°C werden 105 ml einer 0,063 M Lösung von [(4R,5R)-2,2-Dimethyl-1,3-dioxolan-4,5-bis(diphenylmethoxy)]-cyclopentadienyl-chloro-titanat (gemäss Beispiel 2a) in Diethylether zugetropft. Man rührt während 21h bei -78°C und gibt die Lösung anschliessend über eine Stahlkanüle zu einer auf -78°C gekühlten Lösung von 0,47 g Butyraldehyd in 10 ml THF. Nach 23 h Rühren bei -78°C gibt man 2,4 ml Essigsäure zu, rührt 15 min, setzt 12 ml Wasser zu, wärmt auf Raumtemperatur auf, rührt 2h und dampft schonend ein. Der Rückstand wird in 0,1 M HCl aufgenommen und dreimal mit Diethylether exträhiert. Die organischen Phasen werden zweimal mit 0,1 N HCl gewaschen. Die vereinigten wässrigen Phasen stellt man mit 2 N NaOH auf pH 4 ein, dampft sie bis zu einem Restvolumen von etwa 50 ml ein, versetzt den Rückstand mit 40 ml Dioxan, 3,36 g NaHCO₃ und 2,39 g Di-tert.butyl-dicarbonat. Nach 18 h Rühren wird das Reaktionsgemisch mit Diethylether verdünnt und zweimal mit Wasser und gesättigter Kochsalzlösung gewaschen; die Wasserphasen werden zweimal mit Diethylether extrahiert. Die organischen Extrakte werden getrocknet (Na₂SO₄) und eingedampft: 4,37 g Rohprodukt. Eine Probe (5 mg) davon wird in 0,3 ml wasserfreier 2 N HCl in Ethanol während 2h auf 100° erhitzt, eingedampft, mit 0,2 ml Trifluoressigsäureanhydrid in 0,3 ml Methylenchlorid während 2h umgesetzt und durch Kapillar-Gaschromatographie (Chirasil-*L*-Val, 90 - 180°C/2°C pro min.) analysiert. Die Probe besteht demgemäss aus 97,1 % (2S,3R)-Enantiomer (Retentionszeit 11,8 min) und 2,9 % (2R,3S)-Enantiomer (Retentionszeit 10.9 min.). Eine Chromatographie des Rest des Rückstandes (Kieselgel; Essigsäureethylester/Hexan 1:5) liefert 1,03 g (2S,3R)-tert.Butoxycarbonylamino-3-hydroxy-hexansäure-tert.butylester als Oel ([α]_{D}=-10,4°; c=1.3 in Ethanol).

### Beispiel 25: Herstellung von (1S,2S)-2-Methyl-1-phenyl-3-buten-1-ol

0,4 g (0,64mmol) der Titanverbindung hergestellt gemäss Beispiel 4 wird in 40ml Diethylether gelöst, auf -74 °C abgekühlt, mit 0,065ml (0,65mmol) Benzaldehyd versetzt und 2h bei -74°C gerührt. Anschliessend wird die Reaktionslösung mit 20ml einer wässrigen Ammoniumfluoridlösung hydrolisiert,über Nacht bei RT gerührt, über Hyflo filtiert, 3x mit 40ml Diethylether extrahiert, mit einer gesättigten NaCl-Lösung gewaschen, über Na₂SO₄ getrocknet und eingedampft.

Das Produkt wird durch Flash-Chromatographie (60g SiO₂, Diethylether/Hexan 1:5) gereinigt. Ausbeute: 0.082 g (90%) ee = 98%, de > 97%.

### Beispiel 26: Herstellung von (S)-1-Phenylethanol

Eine Lösung von 5mmol der Zirkonverbindung von Beispiel 9b in 60 ml Diethylether wird auf -74°C abgekühlt, mit 0,4ml (4mmol) Benzaldehyd versetzt, 4 Stunden bei dieser Temperatur gerührt und anschliessend mit 30ml einer wässrigen 45%-igen NH₄F-Lösung versetzt. Die beige Suspension wird auf RT erwärmt, weitere 12 Stunden gerührt, genutscht und am Rotavapor eingeengt. Das Rohprodukt (0,81g) wird Flash-chromatographiert (80g Kieselgel, 25cm lang ⌀ 2cm, 30ml/Fraktion, 0,4bar, Diethylether/Hexan 1:5) und im Kugelrohr destilliert. Man erhält 0,303g Produkt Ausbeute: 62%, ee: 97%.

Wird anstatt der Zirkonverbindung die entsprechende Hafniumverbindung von Beispiel 10b verwendet, wird das gleiche Produkt erhalten. Ausbeute: 60%, ee 97%.

## Patentansprüche (Patentansprüche für folgende(n) Vertragsstaat(en): AT, BE, CH, DE, DK, FR, GB, GR, IT, LI, LU, NL, SE)

1. Verbindungen der Formel I 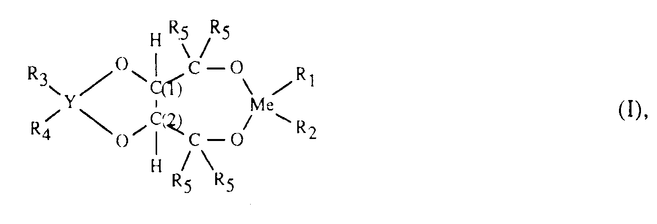 worin
a) Me für vierwertiges Titan steht und R₁ Allyl, 1-Methylallyl, 2-Methylallyl, -CH₂CH=CH-P(C₆H₅), -CH(SO₂R₆)CH=CH-Si(R₆)₃, 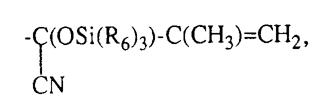 -CH₂-CH=CH-SO₂-R₆, -CH₂-CH=CH-OR' oder 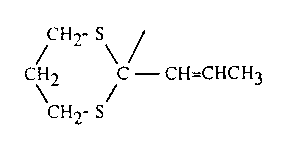 darstellt, R₆ für Phenyl, Benzyl oder C₁-C₈-Alkyl steht und R' C₁-C₈-Alkyl oder -Si(R₆)₃ bedeutet, oder R₁ einen über das O-Atom oder N-Atom gebundenen Rest eines Enols, Enamins oder Enhydrazins mit bis zu 20 C-Atomen bedeutet; oder
b) Me für vierwertiges Zirkonium oder Hafnium steht und R₁ lineares oder verzweigtes C₁-C₁₈-Alkyl, C₂-C₁₂-Alkenyl, C₂-C₁₂-Alkinyl, oder gegebenenfalls mit C₁-C₄-Alkyl substituiertes C₃-C₈-Cycloalkyl oder C₃-C₈-Cycloalkenyl, oder C₆-C₁₂-Aryl, C₇-C₁₆-Alkaryl, C₆-C₁₆-Aralkyl, C₈-C₁₆-Alkaralkyl, C₈-C₁₆-Aralkenyl, C₉-C₁₆-Alkaralkenyl, C₈-C₁₆-Aralkinyl oder C₉-C₁₆-Alkaralkinyl bedeutet, die unsubstituiert oder ein- oder mehrfach mit (C₆H₅)₂P-, (R₆O)₂P(O)-, (R₆)₃Si-, (R₆)₃SiO-, R₆SO₂-, -S-C₂-C₄-Alkylen-S-, -O-C₂-C₄-Alkylen-O-, wobei R₆ für Phenyl, Benzyl oder C₁-C₈-Alkyl steht, Cyano, F, Nitro, C₁-C₁₂-Alkylthio, C₁-C₁₂-Alkoxy, -NR₁₈R₁₉ oder -COR₇, wobei R₇ C₁-C₁₂-Alkoxy ist, R₁₈ und R₁₉ C₁-C₁₂-Alkyl, Cyclopentyl, Cyclohexyl, Phenyl, Benzyl oder (R₆)₃Si oder R₁₈ und R₁₉ zusammen C₃-C₆-Alkylen, 3-Oxapentylen oder -Si(R₆)₂-C₂-C₃-Alkylen-Si(R₆)₂- bedeuten, substituiert sind; oder R₁ einen über das O-Atom oder N-Atom gebundenen Rest eines Enols, Enamins oder Enhydrazins mit bis zu 20 C-Atomen bedeutet;
R₂ gegebenenfalls durch C₁-C₆-Alkyl, C₂-C₆-Alkenyl, C₁-C₆-Alkoxy, C₅- oder C₆-Cycloalkyl, C₆-C₁₂-Aryl, C₇-C₁₃-Aralkyl, Tri-C₁-C₆-alkoxysilyl, Tri-C₁-C₆-alkylsilyl, R₆SO₂- oder Halogen substituiertes Cyclopentadienyl oder Indenyl darstellt,
Y für C, Si oder -Si-O-SiR₃R₄- steht,
R₃ und R₄ unabhängig voneinander H, lineares oder verzweigtes C₁-C₁₈-Alkyl, C₂-C₁₂-Alkenyl, oder gegebenenfalls mit C₁-C₄-Alkyl substituiertes C₃-C₈-Cycloalkyl oder C₃-C₈-Cycloalkenyl, oder C₆-C₁₂-Aryl, C₇-C₁₆-Aralkyl, C₇-C₁₆-Alkaryl, C₈-C₁₆-Alkaralkyl, C₈-C₁₆-Aralkenyl oder C₉-C₁₆-Alkaralkenyl oder R₃ und R₄ zusammen -CₙH₂ₙ- mit n gleich 3 bis 7 bedeuten, die unsubstituiert oder mit -CN, -F, -Cl, -Br, -Nitro, C₁-C₁₂-Alkylthio oder C₁-C₁₂-Alkoxy substituiert sind,
R₅ unsubstituiertes oder mit C₁-C₁₂-Alkyl, C₁-C₁₂-Alkoxy, C₁-C₁₂-Alkylthio, Cyano, Di(C₁-C₄-Alkyl)amino, Di(C₁-C₄-Alkyl)aminocarbonyl, Fluor, C₆-C₁₀-Aryl, R₆SO₂-, C₁-C₁₂-Alkoxymethyl, C₁-C₁₂-Alkylthiomethyl, Di(C₁-C₄-Alkyl)aminomethyl oder Di(C₁-C₄-Alkyl)aminocarbonylmethyl substituiertes C₆-C₁₀-Aryl, Heteroaryl, Benzyl, Naphthylmethyl oder Heteroarylalkyl darstellt, wobei das Heteroaryl 5- oder 6-gliedrig ist und ein Heteroatom aus der Gruppe O, S und N enthält, oder C₁-C₁₂-Alkyl, C₂-C₁₂-Alkenyl oder -Alkinyl, C₃-C₈-Cycloalkyl oder -Cycloalkenyl bedeutet,
und C(1) und C(2) chirale C-Atome sind, in Form von überwiegend Enantiomeren oder Diastereomeren.

2. Verbindungen gemäss Anspruch 1, worin R₁ gegebenenfalls ein- oder mehrfach mit R₁₈R₁₉N-, Cyano, Nitro, C₁-C₆-Alkylthio, C₁-C₆-Alkoxy oder -COR₇, worin R₇ C₁-C₁₂-Alkoxy ist, substituiertes lineares oder verzweigtes C₁-C₁₂-Alkyl, C₂-C₁₂-Alkenyl, C₂-C₁₂-Alkinyl, Cycloalkyl oder Cycloalkenyl mit 3 bis 8 Ring-C-Atomen, C₆-C₁₂-Aryl, C₇-C₁₆-Alkaryl oder -Aralkyl, C₈-C₁₆- Alkaralkyl, C₈-C₁₆-Aralkenyl, C₉-C₁₆-Alkaralkenyl, C₈-C₁₆-Aralkinyl oder C₉-C₁₆-Alkaralkinyl; oder ein über das Enolsauerstoffatom oder über das Enaminstickstoffatom gebundener Rest eine Enols, Enamins oder Enhydrazins mit bis zu 20 C-Atomen ist.

3. Verbindungen gemäss Anspruch 2, worin R₁ gegebenenfalls ein- oder mehrfach mit R₁₈R₁₉N-, Cyano, Nitro, C₁-C₆-Alkoxy, C₁-C₆-Alkylthio oder -COR₇, worin R₇ C₁-C₁₂-Alkoxy ist, substituiertes lineares oder verzweigtes C₁-C₆-Alkyl, C₂-C₆-Alkenyl, C₂-C₆-Alkinyl, Cycloalkyl oder Cycloalkenyl mit 3 bis 6 Ring-C-Atomen, Phenyl, (C₁-C₁₀-Alkyl)phenyl, Phenyl(C₁-C₂-alkyl), (C₁-C₈-Alkyl)phenyl(C₁-C₂-alkyl), Phenylvinyl, Phenylethinyl oder Phenylpropargyl, (C₁-C₈-Alkyl)phenylvinyl, (C₁-C₈-Alkyl)phenylethinyl oder (C₁-C₇-Alkyl)phenylpropargyl; oder ein über ein Enolsauerstoffatom oder Enaminostickstoffatom gebundener Rest eines Enols, Enamins oder Enhydrazins mit bis zu 20 C-Atomen bedeutet.

4. Verbindungen gemäss Anspruch 2, worin R₁ gegebenenfalls ein- oder mehrfach mit R₁₈R₁₉N-, Cyano, Nitro, C₁-C₆-Alkoxy, C₁-C₆-Alkylthio oder -COR₇, worin R₇ C₁-C₁₂-Alkoxy ist, substituiertes Methyl, Ethyl, Vinyl, Allyl, Crotyl, Ethinyl, Propargyl, Cyclopentyl, Cyclohexyl, Cyclopentenyl und Cyclohexenyl, Phenyl, Methylphenyl, Benzyl, 1-Phenyleth-2-yl, Methylbenzyl, Phenylvinyl, Methylphenylvinyl, Phenylethinyl, Phenylpropargyl, Methylphenylethinyl, Dimethylphenylethinyl oder -propargyl; oder ein über das Enolsauerstoffatom oder über das Enaminostickstoffatom gebundener Rest eines Enols, Enamins oder Enhydrazins mit 2 bis 16 C-Atomen bedeutet.

5. Verbindungen gemäss Anspruch 1, worin R₂ gegebenenfalls mit C₁-C₆- Alkyl, C₂-C₆-Alkenyl, C₁-C₆-Alkoxy, Cycloalkyl mit 5 oder 6 Ring-C-Atomen, Phenyl, Benzyl, Trialkoxysilyl mit 1 bis 6 C-Atomen in den Alkoxygruppen, Trialkylsilyl mit 1 bis 6 C-Atomen in den Alkylgruppen, F, Cl, Br, oder R₆SO₂- mit R₆ gleich Phenyl, Tolyl, Benzyl oder C₁-C₆-Alkyl substituiertes Cyclopentadienyl oder Indenyl darstellt.

6. Verbindungen gemäss Anspruch 5, worin R₂ gegebenenfalls mit C₁-C₆-Alkyl oder Trimethylsilyl substituiertes Cyclopentadienyl darstellt.

7. Verbindungen gemäss Anspruch 1, worin R₃ und R₄ unabhängig voneinander H, C₁-C₁₂-Alkyl, C₂-C₁₂-Alkenyl, gegebenenfalls mit C₁-C₄-Alkyl substituiertes Cycloalkyl oder Cycloalkenyl mit 5 oder 6 Ring-C-Atomen, Phenyl, Naphthyl, Benzyl, C₁-C₆-Alkylphenyl oder C₁-C₆-Alkylbenzyl oder R₃ und R₄ zusammen -CₙH₂ₙ- mit n gleich 4 bis 6 darstellen, die unsubstituiert oder mit -CN, -F, -Cl, -Nitro, C₁-C₆-Alkylthio oder C₁-C₆-Alkoxy substituiert sind.

8. Verbindungen gemäss Anspruch 7, worin R₃ und R₄ unabhängig voneinander H, C₁-C₆-Alkyl, gegebenenfalls mit C₁-C₄-Alkyl substituiertes Cyclopentyl oder Cyclohexyl, Phenyl, Benzyl, C₁-C₆-Alkylphenyl oder C₁-C₆-Alkylbenzyl oder R₃ und R₄ zusammen Penta- oder Tetramethylen darstellen.

9. Verbindungen gemäss Anspruch 1, worin R₃ H und R₄ oder R₃ und R₄ ungleich H sind.

10. Verbindungen gemäss Anspruch 1, worin Y für C steht.

11. Verbindungen gemäss Anspruch 1, worin R₅ C₂-C₄-Alkenyl, 2-Furyl oder C₆-C₁₀-Aryl ist, das unsubstituiert oder mit C₁-C₆-Alkyl oder F substituiert ist.

12. Verbindungen gemäss Anspruch 11, worin das Aryl Phenyl, Methylphenyl, Pentafluorphenyl oder Naphthyl ist.

13. Verbindungen gemäss Anspruch 11, worin R₅ Propenyl, Phenyl, Pentafluorphenyl, Methylphenyl oder Furyl ist.

14. Verbindungen gemäss Anspruch 1, worin
R₁ lineares oder verzweigtes C₁-C₄-Alkyl, C₂-C₆-Alkenyl, oder gegebenenfalls mit C₁-C₄-Alkyl substituiertes Cycloalkyl oder Cycloalkenyl mit 3 bis 6 Ring-C-Atomen, oder Phenyl, Benzyl, C₁-C₆-Alkylphenyl oder C₁-C₆-Alkylbenzyl darstellt, das unsubstituiert oder mit Sekundäramino, Cyano, Nitro, C₁-C₆-Alkoxy, C₁-C₆-Alkylthio oder -COR₇, worin R₇ C₁-C₁₂-Alkoxy ist, darstellt, oder
R₁ einen über ein Enolsauerstoffatom oder ein Enaminostickstoffatom gebundener Rest eines Enols, Enamins oder Enhydrazins mit bis zu 12 C-Atomen bedeutet,
R₂ Cyclopentadienyl ist, das unsubstituiert oder mit C₁-C₄-Alkyl oder Trimethylsilyl substituiert ist,
Y für C steht,
R₃ H ist oder die Bedeutung von R₄ hat und
R₄ C₁-C₁₂-Alkyl, Phenyl, Benzyl, Cyclopentyl, Cyclohexyl darstellt oder
R₃ und R₄ zusammen Tetra- oder Pentamethylen bedeuten,
R₅ für C₂-C₄-Alkenyl, Phenyl, Pentafluorphenyl oder Furyl steht, und
Me vierwertiges Titan, Zirkonium oder Hafnium bedeutet.

15. Verbindungen gemäss Anspruch 1, worin die C(1)- und C(2)-Atome in Formel I entweder R, R- oder S,S-Konfiguration aufweisen.

16. Verbindungen gemäss Anspruch 15, worin Y ein chirales C-Atom ist, in Form der Diastereomeren.

17. Verbindungen gemäss Anspruch 1, worin R₁ C₁-C₄-Alkyl, Allyl oder der Rest eines Esterenolats mit 2 bis 12 C-Atomen ist, das mit R₁₈R₁₉N- substituiert sein kann, R₂ gegebenenfalls mit Methyl oder Trimethylsilyl substituiertes Cyclopentadienyl bedeutet, R₃ H darstellt oder die Bedeutung von R₄ hat und R₄ C₁-C₆-Alkyl ist, R₅ Propenyl, Phenyl, Pentafluorphenyl oder Furyl bedeutet und Me für vierwertiges Titan, Zirkonium oder Hafnium und Y für C stehen.

18. Verfahren zur Herstellung von Verbindungen der Formel I gemäss Anspruch 1, dadurch gekennzeichnet, dass man eine Verbindung der Formel II 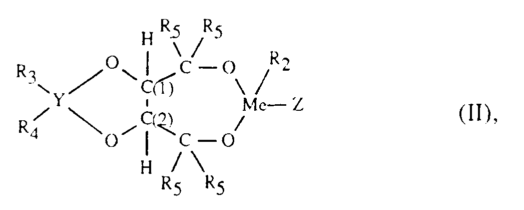 mit einer Verbindung der Formel III
R₁^{⊖}-M^{⊕} (III)
in Gegenwart eines inerten Lösungsmittels und inerten Schutzgases umsetzt, wobei R₁, R₂, R₃, R₄, R₅, Me und Y die in Anspruch 1 angegebenen Bedeutungen haben, Z für ein Anion steht und M^{⊕} für Li^{⊕}, Na^{⊕}, K^{⊕}, MgX^{⊕}, ZnX^{⊕}, CdX^{⊕}, HgX^{⊕}, CuX^{⊕} oder quaternäres Ammonium steht, wobei X Halogen bedeutet.

19. Verbindungen der Formel II, 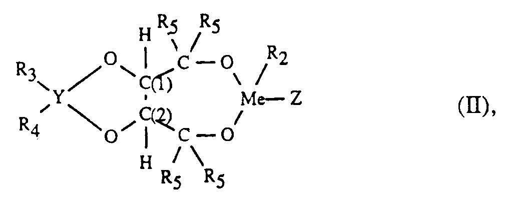 worin R₂, R₃, R₄, R₅ Y und Me die in Anspruch 1 angegebenen Bedeutungen haben und Z für ein Anion steht.

20. Verwendung von Verbindungen der Formel I gemäss Anspruch 1 als enantioselektive Reaktanden für Verbindungen mit Aldheyd-, Keto- und/oder N-substituierten Imingrupen.

21. Verfahren zur Herstellung von sekundären und tertiären Alkoholen und sekundären Aminen, dadurch gekennzeichnet, dass man Aldehyde, Ketone, N-substituierte Aldimine oder Ketimine mit 1 Mol einer Verbindung der Formel I gemäss Anspruch 1 pro Mol Aldehyd-, Keto- oder Imingruppe umsetzt.

22. Verfahren gemäss Anspruch 21, dadurch gekennzeichnet, dass es bei Temperaturen von -80 bis 30°C durchgeführt wird.

## Patentansprüche (Patentansprüche für folgende(n) Vertragsstaat(en): ES)

1. Verfahren zur Herstellung von Verbindungen der Formel I 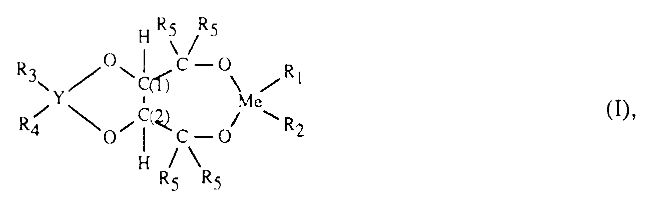 worin
a) Me für vierwertiges Titan steht und R₁ Allyl, 1-Methylallyl, 2-Methylallyl, -CH₂CH=CH-P(C₆H₅), -CH(SO₂R₆)CH=CH-Si(R₆)₃, 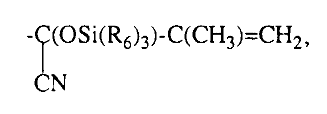 -CH₂-CH=CH-SO₂-R₆, -CH₂-CH=CH-OR' oder 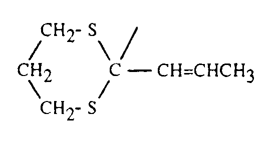 darstellt, R₆ für Phenyl, Benzyl oder C₁-C₈-Alkyl steht und R' C₁-C₈-Alkyl oder -Si(R₆)₃ bedeutet, oder R₁ einen über das O-Atom oder N-Atom gebundenen Rest eines Enols, Enamins oder Enhydrazins mit bis zu 20 C-Atomen bedeutet; oder
b) Me für vierwertiges Zirkonium oder Hafnium steht und R₁ lineares oder verzweigtes C₁-C₁₈-Alkyl, C₂-C₁₂-Alkenyl, C₂-C₁₂-Alkinyl, oder gegebenenfalls mit C₁-C₄-Alkyl substituiertes C₃-C₈-Cycloalkyl oder C₃-C₈-Cycloalkenyl, oder C₆-C₁₂-Aryl, C₇-C₁₆-Alkaryl, C₆-C₁₆-Aralkyl, C₈-C₁₆-Alkaralkyl, C₈-C₁₆-Aralkenyl, C₉-C₁₆-Alkaralkenyl, C₈-C₁₆-Aralkinyl oder C₉-C₁₆-Alkaralkinyl bedeutet, die unsubstituiert oder ein- oder mehrfach mit (C₆H₅)₂P-, (R₆O)₂P(O)-, (R₆)₃Si-, (R₆)₃SiO-, R₆SO₂-, -S-C₂-C₄-Alkylen-S-, -O-C₂-C₄-Alkylen-O-, wobei R₆ für Phenyl, Benzyl oder C₁-C₈-Alkyl steht, Cyano, F, Nitro, C₁-C₁₂-Alkylthio, C₁-C₁₂-Alkoxy, -NR₁₈R₁₉ oder -COR₇, wobei R₇ C₁-C₁₂-Alkoxy ist, R₁₈ und R₁₉ C₁-C₁₂-Alkyl, Cyclopentyl, Cyclohexyl, Phenyl, Benzyl oder (R₆)₃Si oder R₁₈ und R₁₉ zusammen C₃-C₆-Alkylen, 3-Oxapentylen oder -Si(R₆)₂-C₂-C₃-Alkylen-Si(R₆)₂- bedeuten, substituiert sind; oder R₁ einen über das O-Atom oder N-Atom gebundenen Rest eines Enols, Enamins oder Enhydrazins mit bis zu 20 C-Atomen bedeutet;
R₂ gegebenenfalls durch C₁-C₆-Alkyl, C₂-C₆-Alkenyl, C₁-C₆-Alkoxy, C₅- oder C₆-Cycloalkyl, C₆-C₁₂-Aryl, C₇-C₁₃-Aralkyl, Tri-C₁-C₆-alkoxysilyl, Tri-C₁-C₆-alkylsilyl, R₆SO₂- oder Halogen substituiertes Cyclopentadienyl oder Indenyl darstellt,
Y für C, Si oder -Si-O-SiR₃R₄- steht,
R₃ und R₄ unabhängig voneinander H, lineares oder verzweigtes C₁-C₁₈-Alkyl, C₂-C₁₂-Alkenyl, oder gegebenenfalls mit C₁-C₄-Alkyl substituiertes C₃-C₈-Cycloalkyl oder C₃-C₈-Cycloalkenyl, oder C₆-C₁₂-Aryl, C₇-C₁₆-Aralkyl, C₇-C₁₆-Alkaryl, C₈-C₁₆-Alkaralkyl, C₈-C₁₆-Aralkenyl oder C₉-C₁₆-Alkaralkenyl oder R₃ und R₄ zusammen -CₙH₂ₙ- mit n gleich 3 bis 7 bedeuten, die unsubstituiert oder mit -CN, -F, -Cl, -Br, -Nitro, C₁-C₁₂-Alkylthio oder C₁-C₁₂-Alkoxy substituiert sind,
R₅ unsubstituiertes oder mit C₁-C₁₂-Alkyl, C₁-C₁₂-Alkoxy, C₁-C₁₂-Alkylthio, Cyano, Di(C₁-C₄-Alkyl)amino, Di(C₁-C₄-Alkyl)aminocarbonyl, Fluor, C₆-C₁₀-Aryl, R₆SO₂-, C₁-C₁₂-Alkoxymethyl, C₁-C₁₂-Alkylthiomethyl, Di(C₁-C₄-Alkyl)aminomethyl oder Di(C₁-C₄-Alkyl)aminocarbonylmethyl substituiertes C₆-C₁₀-Aryl, Heteroaryl, Benzyl, Naphthylmethyl oder Heteroarylalkyl darstellt, wobei das Heteroaryl 5- oder 6-gliedrig ist und ein Heteroatom aus der Gruppe O, S und N enthält, oder
C₁-C₁₂-Alkyl, C₂-C₁₂-Alkenyl oder -Alkinyl, C₃-C₈-Cycloalkyl oder -Cycloalkenyl bedeutet,
und C(1) und C(2) chirale C-Atome sind, in Form von überwiegend Enantiomeren oder Diastereomeren, dadurch gekennzeichnet, dass man eine Verbindung der Formel II 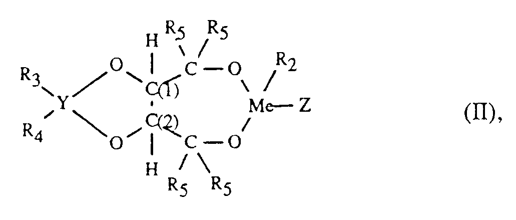 mit einer Verbindung der Formel III
R₁^{⊖}-M^{⊕} (III)
in Gegenwart eines inerten Lösungsmittels und inerten Schutzgases umsetzt, wobei R₁, R₂, R₃, R₄, R₅, Me und Y die zuvor angegebenen Bedeutungen haben, Z für ein Anion steht und M^{⊕} für Li^{⊕}, Na^{⊕}, K^{⊕}, MgX^{⊕}, ZnX^{⊕}, CdX^{⊕}, HgX^{⊕}, CuX^{⊕} oder quaternäres Ammonium steht, wobei X Halogen bedeutet.

2. Verfahren gemäss Anspruch 1, worin R₁ gegebenenfalls ein- oder mehrfach mit R₁₈R₁₉N-, Cyano, Nitro, C₁-C₆-Alkylthio, C₁-C₆-Alkoxy oder -COR₇, worin R₇ C₁-C₁₂-Alkoxy ist, substituiertes lineares oder verzweigtes C₁-C₁₂-Alkyl, C₂-C₁₂-Alkenyl, C₂-C₁₂-Akinyl, Cycloalkyl oder Cycloalkenyl mit 3 bis 8 Ring-C-Atomen, C₆-C₁₂-Aryl, C₇-C₁₆-Alkaryl oder -Aralkyl, C₈-C₁₆- Alkaralkyl, C₈-C₁₆-Aralkenyl, C₉-C₁₆-Alkaralkenyl, C₈-C₁₆-Aralkinyl oder C₉-C₁₆-Alkaralkinyl; oder ein über das Enolsauerstoffatom oder über das Enaminstickstoffatom gebundener Rest eine Enols, Enamins oder Enhydrazins mit bis zu 20 C-Atomen ist.

3. Verfahren gemäss Anspruch 2, worin R₁ gegebenenfalls ein- oder mehrfach mit R₁₈R₁₉N-, Cyano, Nitro, C₁-C₆-Alkoxy, C₁-C₆-Alkylthio oder -COR₇, worin R₇ C₁-C₁₂-Alkoxy ist, substituiertes lineares oder verzweigtes C₁-C₆-Alkyl, C₂-C₆-Alkenyl, C₂-C₆-Alkinyl, Cycloalkyl oder Cycloalkenyl mit 3 bis 6 Ring-C-Atomen, Phenyl, (C₁-C₁₀-Alkyl)phenyl, Phenyl(C₁-C₂-alkyl), (C₁-C₈-Alkyl)phenyl(C₁-C₂-alkyl), Phenylvinyl, Phenylethinyl oder Phenylpropargyl, (C₁-C₈-Alkyl)phenylvinyl, (C₁-C₈-Alkyl)phenylethinyl oder (C₁-C₇-Alkyl)phenylpropargyl; oder ein über ein Enolsauerstoffatom oder Enaminostickstoffatom gebundener Rest eines Enols, Enamins oder Enhydrazins mit bis zu 20 C-Atomen bedeutet.

4. Verfahren gemäss Anspruch 2, worin R₁ gegebenenfalls ein- oder mehrfach mit Sekundäramino, Cyano, Nitro, C₁-C₆-Alkoxy, C₁-C₆-Alkylthio oder -COR₇, worin R₇ C₁-C₁₂-Alkoxy ist, substituiertes Methyl, Ethyl, Vinyl, Allyl, Crotyl, Ethinyl, Propargyl, Cyclopentyl, Cyclohexyl, Cyclopentenyl und Cyclohexenyl, Phenyl, Methylphenyl, Benzyl, 1-Phenyleth-2-yl, Methylbenzyl, Phenylvinyl, Methylphenylvinyl, Phenylethinyl, Phenylpropargyl, Methylphenylethinyl, Dimethylphenylethinyl oder -propargyl; oder ein über das Enolsauerstoffatom oder über das Enaminostickstoffatom gebundener Rest eines Enols, Enamins oder Enhydrazins mit 2 bis 16 C-Atomen bedeutet.

5. Verfahren gemäss Anspruch 1, worin R₂ gegebenenfalls mit C₁-C₆- Alkyl, C₂-C₆-Alkenyl, C₁-C₆-Alkoxy, Cycloalkyl mit 5 oder 6 Ring-C-Atomen, Phenyl, Benzyl, Trialkoxysilyl mit 1 bis 6 C-Atomen in den Alkoxygruppen, Trialkylsilyl mit 1 bis 6 C-Atomen in den Alkylgruppen, F, Cl, Br, oder R₆SO₂- mit R₆ gleich Phenyl, Tolyl, Benzyl oder C₁-C₆-Alkyl substituiertes Cyclopentadienyl oder Indenyl darstellt.

6. Verfahren gemäss Anspruch 5, worin R₂ gegebenenfalls mit C₁-C₆-Alkyl oder Trimethylsilyl substituiertes Cyclopentadienyl darstellt.

7. Verfahren gemäss Anspruch 1, worin R₃ und R₄ unabhängig voneinander H, C₁-C₁₂-Alkyl, C₂-C₁₂-Alkenyl, gegebenenfalls mit C₁-C₄-Alkyl substituiertes Cycloalkyl oder Cycloalkenyl mit 5 oder 6 Ring-C-Atomen, Phenyl, Naphthyl, Benzyl, C₁-C₆-Alkylphenyl oder C₁-C₆-Alkylbenzyl oder R₃ und R₄ zusammen -CₙH₂ₙ- mit n gleich 4 bis 6 darstellen, die unsubstituiert oder mit -CN, -F, -Cl, -Nitro, C₁-C₆-Alkylthio oder C₁-C₆-Alkoxy substituiert sind.

8. Verfahren gemäss Anspruch 7, worin R₃ und R₄ unabhängig voneinander H, C₁-C₆-Alkyl, gegebenenfalls mit C₁-C₄-Alkyl substituiertes Cyclopentyl oder Cyclohexyl, Phenyl, Benzyl, C₁-C₆-Alkylphenyl oder C₁-C₆-Alkylbenzyl oder R₃ und R₄ zusammen Penta- oder Tetramethylen darstellen.

9. Verfahren gemäss Anspruch 1, worin R₃ H und R₄ oder R₃ und R₄ ungleich H sind.

10. Verfahren gemäss Anspruch 1, worin Y für C steht.

11. Verfahren gemäss Anspruch 1, worin R₅ C₂-C₄-Alkenyl, 2-Furyl oder C₆-C₁₀-Aryl ist, das unsubstituiert oder mit C₁-C₆-Alkyl oder F substituiert ist.

12. Verfahren gemäss Anspruch 11, worin das Aryl Phenyl, Methylphenyl, Pentafluorphenyl oder Naphthyl ist.

13. Verfahren gemäss Anspruch 11, worin R₅ Propenyl, Phenyl, Pentafluorphenyl, Methylphenyl oder Furyl ist.

14. Verfahren gemäss Anspruch 1, worin
R₁ lineares oder verzweigtes C₁-C₄-Alkyl, C₂-C₆-Alkenyl, oder gegebenenfalls mit C₁-C₄-Alkyl substituiertes Cycloalkyl oder Cycloalkenyl mit 3 bis 6 Ring-C-Atomen, oder Phenyl, Benzyl, C₁-C₆-Alkylphenyl oder C₁-C₆-Alkylbenzyl darstellt, das unsubstituiert oder mit Sekundäramino, Cyano, Nitro, C₁-C₆-Alkoxy, C₁-C₆-Alkylthio oder -COR₇, worin R₇ C₁-C₁₂-Alkoxy ist, darstellt, oder
R₁ einen über ein Enolsauerstoffatom oder ein Enaminostickstoffatom gebundener Rest eines Enols, Enamins oder Enhydrazins mit bis zu 12 C-Atomen bedeutet,
R₂ Cyclopentadienyl ist, das unsubstituiert oder mit C₁-C₄-Alkyl oder Trimethylsilyl substituiert ist,
Y für C steht,
R₃ H ist oder die Bedeutung von R₄ hat und
R₄ C₁-C₁₂-Alkyl, Phenyl, Benzyl, Cyclopentyl, Cyclohexyl darstellt oder
R₃ und R₄ zusammen Tetra- oder Pentamethylen bedeuten,
R₅ für C₂-C₄-Alkenyl, Phenyl, Pentafluorphenyl oder Furyl steht, und
Me vierwertiges Titan, Zirkonium oder Hafnium bedeutet.

15. Verfahren gemäss Anspruch 1, worin die C(1)- und C(2)-Atome in Formel I entweder R, R- oder S,S-Konfiguration aufweisen.

16. Verfahren gemäss Anspruch 15, worin Y ein chirales C-Atom ist, in Form der Diastereomeren.

17. Verfahren gemäss Anspruch 1, worin R₁ C₁-C₄-Alkyl, Allyl oder der Rest eines Esterenolats mit 2 bis 12 C-Atomen ist, das mit R₁₈R₁₉N- substituiert sein kann, R₂ gegebenenfalls mit Methyl oder Trimethylsilyl substituiertes Cyclopentadienyl bedeutet, R₃ H darstellt oder die Bedeutung von R₄ hat und R₄ C₁-C₆-Alkyl ist, R₅ Propenyl, Phenyl, Pentafluorphenyl oder Furyl bedeutet und Me für vierwertiges Titan, Zirkonium oder Hafnium und Y für C stehen.

18. Verfahren zur Herstellung von Verbindungen der Formel II, 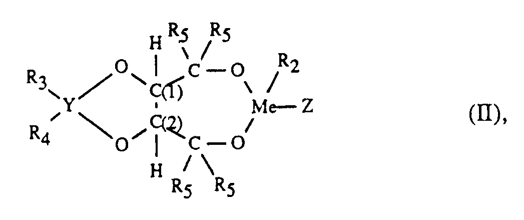 worin R₂, R₃, R₄, R₅ Y und Me die in Anspruch 1 angegebenen Bedeutungen haben und Z für ein Anion steht, dadurch gekennzeichnet, dass man eine Verbindung der Formel
(R₂)MeZ₃,
worin R₂ und Z die in Anspruch 1 angegebenen Bedeutungen haben, mit einer Verbindung der Formel IV 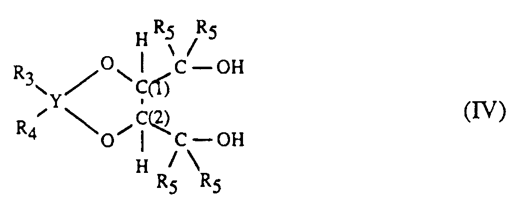 umsetzt.

19. Verwendung von Verbindungen der Formel I gemäss Anspruch 1 als enantioselektive Reaktanden für Verbindungen mit Aldheyd-, Keto- und/oder N-substituierten Imingrupen.

20. Verfahren zur Herstellung von sekundären und tertiären Alkoholen und sekundären Aminen, dadurch gekennzeichnet, dass man Aldehyde, Ketone, N-substituierte Aldimine oder Ketimine mit 1 Mol einer Verbindung der Formel I gemäss Anspruch 1 pro Mol Aldehyd-, Keto- oder Imingruppe umsetzt.

21. Verfahren gemäss Anspruch 20, dadurch gekennzeichnet, dass es bei Temperaturen von -80 bis 30°C durchgeführt wird.

## Claims (Claims for the following Contracting State(s): AT, BE, CH, DE, DK, FR, GB, GR, IT, LI, LU, NL, SE)

1. A compound of the formula I 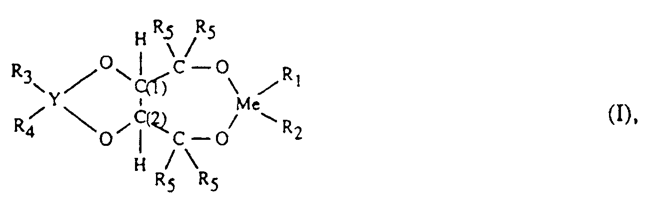 in which
a) Me is tetravalent titanium and R₁ is allyl, 1-methylallyl, 2-methylallyl, -CH₂CH=CH-P(C₆H₅), -CH(SO₂R₆)CH=CH-Si(R₆)₃, 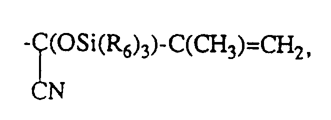 -CH₂-CH=CH-SO₂-R₆, -CH₂-CH=CH-OR' or 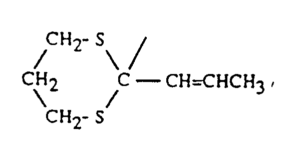 R₆ is phenyl, benzyl or C₁-C₈alkyl and R' C₁-C₈ alkyl or -Si(R₆)₃, or
R₁ is a radical of an enol, enamine or enhydrazine which has up to 20 C atoms and which is attached via the O atom or N atom; or
b) Me is tetravalent zirconium or hafnium and R₁ is linear or branched C₁-C₁₈alkyl, C₂-C₁₂alkenyl, C₂-C₁₂alkynyl or C₃-C₈cycloalkyl or C₃-C₈cycloalkenyl each of which is unsubstituted or substituted by C₁-C₄alkyl, or is C₆-C₁₂aryl, C₇-C₁₆alkaryl, C₆-C₁₆aralkyl, C₈-C₁₆alkaralkyl, C₈-C₁₆aralkenyl, C₉-C₁₆alkaralkenyl, C₈-C₁₆aralkynyl or C₉-C₁₆alkaralkynyl each of which is unsubstituted or monosubstituted or polysubstituted by (C₆H₅)₂P-, (R₆O)₂P(O)-, (R₆)₃Si-, (R₆)₃SiO-, R₆SO₂-, -S-C₂-C₄alkylene-S- or -O-C₂-C₄alkylene-O- in which R₆ is phenyl, benzyl or C₁-C₈alkyl, cyano, F, nitro, C₁-C₁₂alkylthio, C₁-C₁₂alkoxy, -NR₁₈R₁₉ or -COR₇ in which R₇ is C₁-C₁₂alkoxy, R₁₈ and R₁₉ are C₁-C₁₂alkyl, cyclopentyl, cyclohexyl, phenyl, benzyl or (R₆)₃Si, or R₁₈ and R₁₉ together are C₃-C₆alkylene, 3-oxapentylene or -Si(R₆)₂-C₂-C₃alkylene-Si(R₆)₂-; or R₁ is a radical of an enol, enamine or enhydrazine which has up to 20 C atoms and which is attached via the O atom or N atom; R₂ is cyclopentadienyl or indenyl each of which is unsubstituted or substituted by C₁-C₆alkyl, C₂-C₆alkenyl, C₁-C₆alkoxy, C₅ or C₆cycloalkyl, C₆-C₁₂aryl, C₇-C₁₃aralkyl, tri-C₁-C₆-alkoxysilyl, tri-C₁-C₆alkylsilyl, R₆SO₂- or halogen, Y is C, Si or -Si-O-SiR₃R₄-, R₃ and R₄ independently of one another are H, linear or branched C₁-C₁₈alkyl or C₂-C₁₂alkenyl or C₃-C₈cycloalkyl or C₃-C₈cycloalkenyl each of which is unsubstituted or substituted by C₁-C₄alkyl, or are C₆-C₁₂aryl, C₇-C₁₆aralkyl, C₇-C₁₆alkaryl, C₈-C₁₆alkaralkyl, C₈-C₁₆aralkenyl or C₉-C₁₆alkaraldenyl, or R₃ and R₄ together are -CₙH₂ₙ- in which n is 3 to 7 and which is unsubstituted or substituted by -CN, -F, -Cl, -Br, nitro, C₁-C₁₂alkylthio or C₁-C₁₂alkoxy, R₅ is C₆-C₁₀aryl, heteroaryl, benzyl naphthylmethyl or heteroarylalkyl each of which is unsubstituted or substituted by C₁-C₁₂alkyl, C₁-C₁₂alkoxy, C₁-C₁₂alkylthio, cyano, di-(C₁-C₄alkyl)-amino, di-(C₁-C₄alkyl)-aminocarbonyl, fluorine, C₆-C₁₀aryl, R₆SO₂-, C₁-C₁₂alkoxymethyl, C₁-C₁₂alkylthiomethyl, di-(C₁-C₄alkyl)-aminomethyl or di-(C₁-C₄-alkyl)-aminocarbonylmethyl, heteroaryl being 5- or 6-membered and containing a heteroatom from the group O, S and N, or is C₁-C₁₂alkyl, C₂-C₁₂alkenyl, C₂-C₁₂alkynyl, C₃-C₈cycloalkyl or C₃-C₈cycloalkenyl, and C(1) and C(2) are chiral C atoms, predominantly in the form of enantiomers or diastereomers.

2. A compound according to claim 1, in which R₁ is linear or branched C₁-C₁₂alkyl, C₂-C₁₂alkenyl, C₂-C₁₂ alkynyl cycloalkyl or cycloalkenyl each of which has 3 to 8 ring C atoms, C₆-C₁₂aryl, C₇-C₁₆alkaryl, C₇-C₁₆aralkyl, C₈-C₁₆alkaralkyl, C₈-C₁₆aralkenyl, C₉-C₁₆alkaralkenyl, C₈-C₁₆aralkynyl or C₉-C₁₆alkaralkynyl each of which is monosubstituted or polysubstituted by R₁₈R₁₉N-, cyano, nitro, C₁-C₆alkylthio, C₁-C₆alkoxy or -COR₇ in which R₇ is C₁-C₁₂alkoxy; or R₁ is a radical of an enol, enamine or enhydrazine which has up to 20 C atoms and which is attached via the enol oxygen atom or via the enamine nitrogen atom.

3. A compound according to claim 2, in which R₁ is linear or branched C₁-C₆alkyl, C₂-C₆alkenyl, C₂-C₆alkynyl, cycloalkyl or cycloalkenyl each of which has 3 to 6 ring C atoms, phenyl, (C₁-C₁₀alkyl)-phenyl, phenyl-(C₁-C₂alkyl), (C₁-C₈alkyl)-phenyl-(C₁-C₂alkyl), phenylvinyl, phenylethynyl or phenylpropargyl, (C₁-C₈alkyl)-phenylvinyl, (C₁-C₈alkyl)-phenylethynyl or (C₁-C₇alkyl)-phenylpropargyl each of which is monosubstituted or polysubstituted by R₁₈R₁₉N-, cyano, nitro, C₁-C₆alkoxy, C₁-C₆alkylthio or -COR₇ in which R₇ is C₁-C₁₂alkoxy; or R₁ is a radical of an enol, enamine or enhydrazine which has up to 20 C atoms and which is attached via an enol oxygen atom or an enamine nitrogen atom.

4. A compound according to claim 2, in which R₁ is methyl, ethyl, vinyl, allyl, crotyl, ethynyl, propargyl, cyclopentyl, cyclohexyl, cyclopentenyl, cyclohexenyl, phenyl, methylphenyl, benzyl, 1-phenyleth-2-yl, methylbenzyl, phenylvinyl, methylphenylvinyl, phenylethynyl, phenylpropargyl, methylphenylethynyl, dimethylphenylethynyl or dimethylphenylpropargyl each of which is monosubstituted or polysubstituted by R₁₈R₁₉N-, cyano, nitro, C₁-C₆alkoxy, C₁-C₆alkylthio or -COR₇ in which R₇ is C₁-C₁₂alkoxy; or R₁ is a radical of an enol, enamine or enhydrazine which has 2 to 16 C atoms and which is attached via the enol oxygen atom or via the enamino nitrogen atom.

5. A compound according to claim 1, in which R₂ is cyclopentadienyl or indenyl each of which is unsubstituted or substituted by C₁-C₆alkyl, C₂-C₆alkenyl, C₁-C₆alkoxy, cycloalkyl having 5 or 6 ring C atoms, phenyl, benzyl, trialkoxysilyl having 1 to 6 C atoms in the alkoxy groups, trialkylsilyl having 1 to 6 C atoms in the alkyl groups, F, Cl, Br or R₆SO₂- in which R₆ is phenyl, tolyl, benzyl or C₁-C₆alkyl.

6. A compound according to claim 5, in which R₂ is cyclopentadienyl which is unsubstituted or substituted by C₁-C₆alkyl or trimethylsilyl.

7. A compound according to claim 1, in which R₃ and R₄ independently of one another are H, C₁-C₁₂alkyl, C₂-C₁₂alkenyl, cycloalkyl or cycloalkenyl each of which has 5 or 6 ring C atoms and is unsubstituted or substituted by C₁-C₄alkyl, or are phenyl, naphthyl, benzyl, C₁-C₆alkylphenyl or C₁-C₆alkylbenzyl or R₃ and R₄ together are -CₙH₂ₙ- in which n is 4 to 6 and which is un substituted or substituted by -CN, -F, -Cl, nitro, C₁-C₆alkylthio or C₁-C₆alkoxy.

8. A compound according to claim 7, in which R₃ and R₄ independently of one another are H, C₁-C₆alkyl, cyclopentyl or cyclohexyl each of which is unsubstituted or substituted by C₁-C₄alkyl, or are phenyl, benzyl, C₁-C₆alkylphenyl or C₁-C₆alkylbenzyl or R₃ and R₄ together are pentamethylene or tetramethylene.

9. A compound according to claim 1, in which R₃ is H and R₄ or R₃ and R₄ are other than H.

10. A compound according to claim 1, in which Y is C.

11. A compound according to claim 1, in which R₅ is C₂-C₄alkenyl, 2 or C₆-C₁₀aryl each of which is unsubstituted or substituted by C₁-C₆alkyl or F.

12. A compound according to claim 11, in which the aryl is phenyl, methylphenyl, pentafluorophenyl or naphthyl.

13. A compound according to claim 11, in which R₅ is propenyl, phenyl, pentafluorophenyl, methylphenyl or furyl.

14. A compound according to claim 1, in which R₁ is linear or branched C₁-C₄alkyl, C₂-C₆alkenyl or cycloalkyl or cycloalkenyl each of which has 3 to 6 ring C atoms and is unsubstituted or substituted by C₁-C₄alkyl, or R₁ is phenyl, benzyl, C₁-C₆alkylphenyl or C₁-C₆alkylbenzyl each of which is unsubstituted or substituted by secondary amino, cyano, nitro, C₁-C₆alkoxy, C₁-C₆alkylthio or -COR₇ in which R₇ is C₁-C₁₂alkoxy, or R₁ is a radical of an enol, enamine or enhydrazine which has up to 12 C atoms and which is attached via an enol oxygen atom or an enamino nitrogen atom, R₂ is cyclopentadienyl which is unsubstituted or substituted by C₁-C₄alkyl or trimethylsilyl, Y is C, R₅ is H or is as defined for R₄ and R₄ is C₁-C₁₂alkyl, phenyl, benzyl, cyclopentyl or cyclohexyl or R₃ and R₄ together are tetramethylene or pentamethylene, R₅ is C₂-C₄alkenyl, phenyl, pentafluorophenyl or furyl and Me is tetravalent titanium, zirconium or hafnium.

15. A compound according to claim 1, in which the C(1) and C(2) atoms in formula I have either the R,R-configuration or the S,S-configuration.

16. A compound according to claim 15, in which Y is a chiral C atom, in the form of the diastereomers.

17. A compound according to claim 1, in which R₁ is C₁-C₄alkyl, allyl or the radical of an ester-enolate which has 2 to 12 C atoms and can be substituted by R₁₈R₁₉N-, R₂ is cyclopentadienyl which is unsubstituted or substituted by methyl or trimethylsilyl, R₃ is H or is as defined for R₄ and R₄ is C₁-C₆alkyl, R₅ is propenyl, phenyl, pentafluorophenyl or furyl and Me is tetravalent titanium, zirconium or hafnium and Y is C.

18. A process for the preparation of a compound of the formula I according to claim 1, which comprises reacting a compound of the formula II 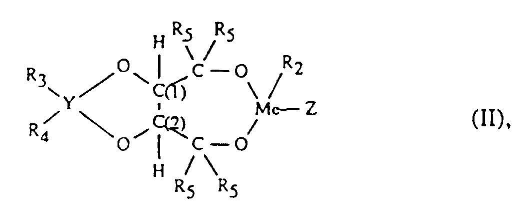 with a compound of the formula III
R₁^{⊖}-M^{⊕} (III)
in the presence of an inert solvent and an inert protective gas, R₁, R₂, R₃, R₄, R₅, Me and Y being as defined in claim 1, Z being an anion and M^{⊕} being Li^{⊕}, Na^{⊕}, K^{⊕}, MgX^{⊕}, ZnX^{⊕}, CdX^{⊕}, HgX^{⊕}, CuX^{⊕} or quaternary ammonium, X being halogen.

19. A compound of the formula II 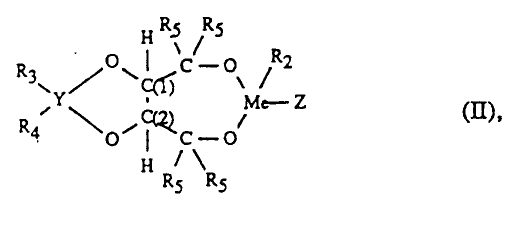 in which R₂, R₃, R₄, R₅, Y and Me are as defined in claim 1 and Z is an anion.

20. The use of a compound of the formula I according to claim 1 as an enantioselective reactant for compounds containing aldehyde, keto and/or N-substituted imine groups.

21. A process for the preparation of secondary and tertiary alcohols and secondary amines, which comprises reacting aldehydes, ketones or N-substituted aldimines or ketimines with 1 mole of a compound of the formula I according to claim 1 per mole of aldehyde, keto or imine group.

22. A process according to claim 21, which is carried out at temperatures from -80 to 30°C.

## Claims (Claims for the following Contracting State(s): ES)

1. A process for the preparation of a compound of the formula I 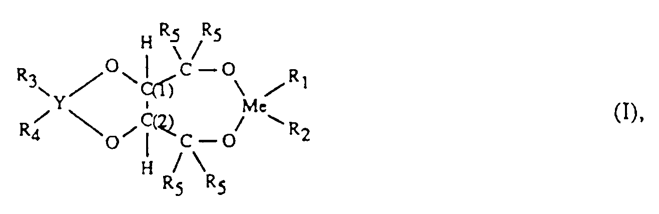 in which
a) Me is tetravalent titanium and R₁ is allyl, 1-methylallyl, 2-methylallyl, -CH₂CH=CH-P(C₆H₅), -CH(SO₂R₆)CH=CH-Si(R₆)₃, 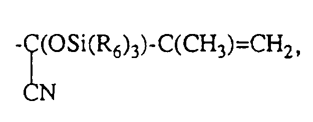 -CH₂-CH=CH-SO₂-R₆, -CH₂-CH=CH-OR' or 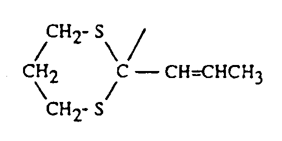 R₆ is phenyl, benzyl or C₁-C₈alkyl and R' is C₁-C₈ alkenyl or
-Si(R₆)₃, or R₁ is a radical of an enol, enamine or enhydrazine which has up to 20 C atoms and which is attached via the O atom or N atom; or
b) Me is tetravalent zirconium or hafnium and R₁ is linear or branched C₁-C₁₈alkyl, C₂-C₁₂alkenyl, C₂-C₁₂alkynyl or C₃-C₈cycloalkyl or C₃-C₈cycloalkenyl each of which is unsubstituted or substituted by C₁-C₄alkyl, or is C₆-C₁₂aryl, C₇-C₁₆alkaryl, C₆-C₁₆aralkyl, C₈-C₁₆alkaralkyl, C₈-C₁₆aralkenyl, C₉-C₁₆alkaralkenyl, C₈-C₁₆aralkynyl or C₉-C₁₆alkaralkynyl each of which is unsubstituted or monosubstituted or polysubstituted by (C₆H₅)₂P-, (R₆O)₂P(O)-, (R₆)₃Si-, (R₆)₃SiO-, R₆SO₂-, -S-C₂-C₄alkylene-S- or -O-C₂-C₄alkylene-O- in which R₆ is phenyl, benzyl or C₁-C₈alkyl, cyano, F, nitro, C₁-C₁₂alkylthio, C₁-C₁₂alkoxy, -NR₁₈R₁₉ or -COR₇ in which R₇ is C₁-C₁₂alkoxy, R₁₈ and R₁₉ are C₁-C₁₂alkyl, cyclopentyl, cyclohexyl, phenyl, benzyl or (R₆)₃Si, or R₁₈ and R₁₉ together are C₃-C₆alkylene, 3-oxapentylene or -Si (R₆)₂-C₂-C₃alkylene-Si(R₆)₂-; or R₁ is a radical of an enol, enamine or enhydrazine which has up to 20 C atoms and which is attached via the O atom or N atom; R₂ is cyclopentadienyl or indenyl each of which is unsubstituted or substituted by C₁-C₆alkyl, C₂-C₆alkenyl, C₁-C₆alkoxy, C₅ or C₆cycloalkyl, C₆-C₁₂aryl, C₇-C₁₃aralkyl, tri-C₁-C₆-alkoxysilyl, tri-C₁-C₆alkylsilyl, R₆SO₂- or halogen, Y is C, Si or -Si-O-SiR₃R₄-, R₃ and R₄ independently of one another are H, linear or branched C₁-C₁₈alkyl or C₂-C₁₂alkenyl or C₃-C₈cycloalkyl or C₃-C₈cycloalkenyl each of which is unsubstituted or substituted by C₁-C₄alkyl, or are C₆-C₁₂aryl, C₇-C₁₆aralkyl, C₇-C₁₆alkaryl, C₈-C₁₆alkaralkyl, C₈-C₁₆aralkenyl or C₉-C₁₆alkaraldenyl, or R₃ and R₄ together are -CₙH₂ₙ- in which n is 3 to 7 and which is unsubstituted or substituted by -CN, -F, -Cl, -Br, nitro, C₁-C₁₂alkylthio or C₁-C₁₂alkoxy, R₅ is C₆-C₁₀aryl, heteroaryl, benzyl naphthylmethyl or heteroarylalkyl each of which is unsubstituted or substituted by C₁-C₁₂alkyl, C₁-C₁₂alkoxy, C₁-C₁₂alkylthio, cyano, di-(C₁-C₄alkyl)-amino, di-(C₁-C₄alkyl)-aminocarbonyl, fluorine, C₆-C₁₀aryl, R₆SO₂-, C₁-C₁₂alkoxymethyl, C₁-C₁₂alkylthiomethyl, di-(C₁-C₄alkyl)-aminomethyl or di-(C₁-C₄-alkyl)-aminocarbonylmethyl, heteroaryl being 5- or 6-membered and containing a heteroatom from the group O, S and N, or is C₁-C₁₂alkyl, C₂-C₁₂alkenyl, C₂-C₁₂alkynyl, C₃-C₈cycloalkyl or C₃-C₈cycloalkenyl,
and C(1) and C(2) are chiral C atoms, predominantly in the form of enantiomers or diastereomers, which comprises reacting a compound of the formula II 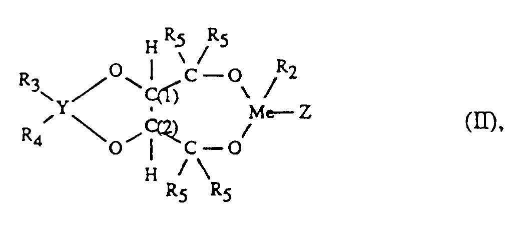 with a compound of the formula III
R₁^{⊖}-M^{⊕} (III)
in the presence of an inert solvent and an inert protective gas, R₁, R₂, R₃, R₄, R₅, Me and Y being as defined above, Z being an anion and M^{⊕} being Li^{⊕}, Na^{⊕}, K^{⊕}, MgX^{⊕}, ZnX^{⊕}, CdX^{⊕}, HgX^{⊕}, CuX^{⊕} or quaternary ammonium, X being halogen.

2. A process according to claim 1, in which R₁ is linear or branched C₁-C₁₂alkyl, C₂-C₁₂alkenyl, C₂-C₁₂alkynyl, cycloalkyl or cycloalkenyl each of which has 3 to 8 ring C atoms, C₆-C₁₂aryl, C₇-C₁₆alkaryl, C₇-C₁₆aralkyl, C₈-C₁₆ alkaralkyl, C₈-C₁₆aralkenyl, C₉-C₁₆alkaralkenyl, C₈-C₁₆aralkynyl or C₉-C₁₆alkaralkynyl each of which is monosubstituted or polysubstituted by R₁₈R₁₉N-, cyano, nitro, C₁-C₆alkylthio, C₁-C₆alkoxy or -COR₇ in which R₇ is C₁-C₁₂alkoxy; or R₁ is a radical of an enol, enamine or enhydrazine which has up to 20 C atoms and which is attached via the enol oxygen atom or via the enamine nitrogen atom.

3. A process according to claim 2, in which R₁ is linear or branched C₁-C₆alkyl, C₂-C₆alkenyl, C₂-C₆alkynyl, cycloalkyl or cycloalkenyl each of which has 3 to 6 ring C atoms, phenyl, (C₁-C₁₀alkyl)-phenyl, phenyl-(C₁-C₂alkyl), (C₁-C₈alkyl)-phenyl-(C₁-C₂alkyl), phenylvinyl, phenylethynyl or phenylpropargyl, (C₁-C₈alkyl)-phenylvinyl, (C₁-C₈alkyl)-phenylethynyl or (C₁-C₇alkyl)-phenylpropargyl each of which is monosubstituted or polysubstituted by R₁₈R₁₉N-, cyano, nitro, C₁-C₆alkoxy, C₁-C₆alkylthio or -COR₇ in which R₇ is C₁-C₁₂alkoxy; or R₁ is a radical of an enol, enamine or enhydrazine which has up to 20 C atoms and which is attached via an enol oxygen atom or an enamine nitrogen atom.

4. A process according to claim 2, in which R₁ is methyl, ethyl, vinyl, allyl, crotyl, ethynyl, propargyl, cyclopentyl, cyclohexyl, cyclopentenyl and cyclohexenyl, phenyl, methylphenyl, benzyl, 1-phenyleth-2-yl, methylbenzyl, phenylvinyl, methylphenylvinyl, phenylethynyl, phenylpropargyl, methylphenylethynyl, dimethylphenylethynyl or dimethylphenylpropargyl each of which is monosubstituted or polysubstituted by secondary amino, cyano, nitro, C₁-C₆alkoxy, C₁-C₆alkylthio or -COR₇ in which R₇ is C₁-C₁₂alkoxy; or R₁ is a radical of an enol, enamine or enhydrazine which has 2 to 16 C atoms and which is attached via the enol oxygen atom or via the enamino nitrogen atom.

5. A process according to claim 1, in which R₂ is cyclopentadienyl or indenyl each of which is unsubstituted or substituted by C₁-C₆alkyl, C₂-C₆alkenyl, C₁-C₆alkoxy, cycloalkyl having 5 or 6 ring C atoms, phenyl, benzyl, trialkoxysilyl having 1 to 6 C atoms in the alkoxy groups, trialkylsilyl having 1 to 6 C atoms in the alkyl groups, F, Cl, Br or R₆SO₂- in which R₆ is phenyl, tolyl, benzyl or C₁-C₆alkyl.

6. A process according to claim 5, in which R₂ is cyclopentadienyl which is unsubstituted or substituted by C₁-C₆alkyl or trimethylsilyl.

7. A process according to claim 1, in which R₃ and R₄ independently of one another are H, C₁-C₁₂alkyl, C₂-C₁₂alkenyl, cycloalkyl or cycloalkenyl each of which has 5 or 6 ring C atoms and is unsubstituted or substituted by C₁-C₄alkyl, or are phenyl, naphthyl, benzyl, C₁-C₆alkylphenyl or C₁-C₆alkylbenzyl or R₃ and R₄ together are -CₙH₂ₙ- in which n is 4 to 6 and which is unsubstituted or substituted by -CN, -F, -Cl, nitro, C₁-C₆alkylthio or C₁-C₆alkoxy.

8. A process according to claim 7, in which R₃ and R₄ independently of one another are H, C₁-C₆alkyl, cyclopentyl or cyclohexyl each of which is unsubstituted or substituted by C₁-C₄alkyl, or are phenyl, benzyl, C₁-C₆alkylphenyl or C₁-C₆alkylbenzyl or R₃ and R₄ together are pentamethylene or tetramethylene.

9. A process according to claim 1, in which R₃ is H and R₄ or R₃ and R₄ are other than H.

10. A process according to claim 1, in which Y is C.

11. A process according to claim 1, in which R₅ is C₂-C₄alkenyl, 2-furyl or C₆-C₁₀aryl each of which is unsubstituted or substituted by C₁-C₆alkyl or F.

12. A process according to claim 11, in which the aryl is phenyl, methylphenyl, pentafluorophenyl or naphthyl.

13. A process according to claim 11, in which R₅ is propenyl, phenyl, pentafluorophenyl, methylphenyl or furyl.

14. A process according to claim 1, in which R₁ is linear or branched C₁-C₄alkyl, C₂-C₆alkenyl or cycloalkyl or cycloalkenyl each of which has 3 to 6 ring C atoms and is unsubstituted or substituted by C₁-C₄alkyl, or R₁ is phenyl, benzyl, C₁-C₆alkylphenyl or C₁-C₆alkylbenzyl each of which is unsubstituted or substituted by secondary amino, nyano, nitro, C₁-C₆alkoxy, C₁-C₆alkylthio or -COR₇ in which R₇ is C₁-C₁₂alkoxy, or R₁ is a radical of an enol, enamine or enhydrazine which has up to 12 C atoms and which is attached via an enol oxygen atom or an enamino nitrogen atom, R₂ is cyclopentadienyl which is unsubstituted or substituted by C₁-C₄alkyl or trimethylsilyl, Y is C, R₅ is H or is as defined for R₄ and R₄ is C₁-C₁₂alkyl, phenyl, benzyl, cyclopentyl or cyclohexyl or R₃ and R₄ together are tetramethylene or pentamethylene, R₅ is C₂-C₄alkenyl, phenyl, pentafluorophenyl or furyl and Me is tetravalent titanium, zirconium or hafnium.

15. A process according to claim 1, in which the C(1) and C(2) atoms in formula I have either the R,R-configuration or the S,S-configuration.

16. A process according to claim 15, in which Y is a chiral C atom, in the form of the diastereomers.

17. A process according to claim 1, in which R₁ is C₁-C₄alkyl, allyl or the radical of an ester-enolate which has 2 to 12 C atoms and can be substituted by R₁₈R₁₉N-, R₂ is cyclopentadienyl which is unsubstituted or substituted by methyl or trimethylsilyl, R₃ is H or is as defined for R₄ and R₄ is C₁-C₆alkyl, R₅ is propenyl, phenyl, pentafluorophenyl or furyl and Me is tetravalent titanium, zirconium or hafnium and Y is C.

18. A process for the preparation of a compound of the formula II 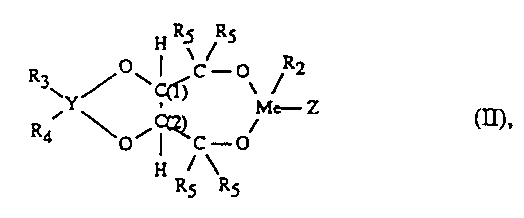 in which R₂, R₃, R₄, R₅, Y and Me are as defined in claim 1 and Z is an anion, which comprises reacting a compound of the formula
(R₂)MeZ₃
in which R₂ and Z are as defined in claim 1 with a compound of the formula IV

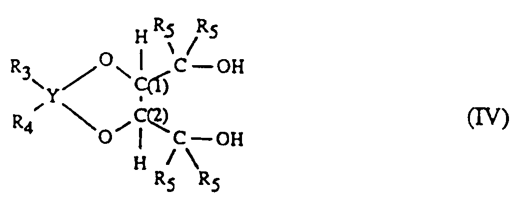

19. The use of a compound of the formula I according to claim 1 as an enantioselective reactant for compounds containing aldehyde, keto and/or N-substituted imine groups.

20. A process for the preparation of secondary and tertiary alcohols and secondary amines, which comprises reacting aldehydes, ketones or N-substituted aldimines or ketimines with 1 mole of a compound of the formula I according to claim 1 per mole of aldehyde, keto or imine group.

21. A process according to claim 20, which is carried out at temperatures from -80 to 30°C.

## Revendications (Revendications pour l'(les) Etat(s) contractant(s) suivant(s): AT, BE, CH, DE, DK, FR, GB, GR, IT, LI, LU, NL, SE)

1. Composés de formule I 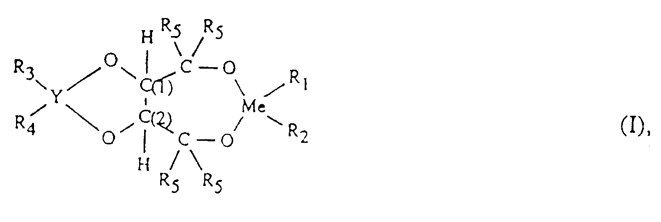 dans laquelle
a) Me représente un titane tétravalent et R₁ représente les allyle, 1-méthylallyle, 2-méthylallyle -CH₂CH=CH-P(C₆H₅), -CH(SO₂R₆)CH=CH-Si(R₆)₃, 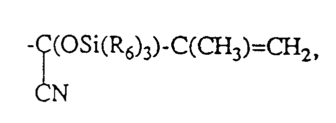 -CH₂-CH=CH-SO₂-R₆, -CH₂-CH=CH-OR' ou 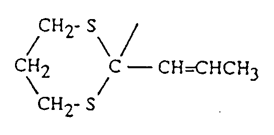 R₆, représente un phényle, un benzyle ou un alkyle en C₁-C₈ et R' signifie un alkyle en C₁-C₈ ou un -Si(R₆)₃, ou R₁ est un reste d'un énol, d'une énamine ou d'une ène-hydrazine ayant jusqu'à 20 atomes de carbone et étant lié par l'atome d'oxygène ou d'azote; ou
b) Me représente un zirconium ou un hafnium tétravalent et R₁ signifie un alkyle en C₁-C₁₈ linéaire ou ramifié, un alcényle en C₂-C₁₂, un alcynyle en C₂-C₁₂, ou un cycloalkyle en C₃-C₈ éventuellement substitué par des alkyle en C₁-C₄ ou un cycloalcényle en C₃-C₈, ou un aryle en C₆-C₁₂, un alkaryle en C₇-C₁₆, un aralkyle en C₆-C₁₆, un alkaralkyle en C₈-C₁₆, un aralcényle en C₈-C₁₆, un alkaralcényle en C₉-C₁₆, un aralcynyle en C₈-C₁₆ ou un alkaralcynyle en C₉-C₁₆, qui sont non substitués ou substitués une plusieurs fois par les (C₆H₅)₂P-, (R₆O)₂P(O)-, (R₆)₃Si-, (R₆)₃SiO-, R₆SO₂-, -S-(alkylène en C₂-C₄)-S-, -O-(alkylène en C₂-C₄)-O-, R₆ représentant un phényle, un benzyle ou un alkyle en C₁-C₈, cyano, F, nitro, alkylthio en C₁-C₁₂, alcoxy en C₁-C₁₂, -NR₁₈R₁₉ ou -COR₇, R₇ étant un alcoxy en C₁-C₁₂, R₁₈ et R₁₉ signifiant un alkyle en C₁-C₁₂, un cyclopentyle, un cyclohexyle, un phényle, un benzyle ou (R₆)₃Si ou R₁₈ et R₁₉ ensemble signifient un alkylène en C₃-C₆, 3-oxapentylène ou un -Si(R₆)₂-(alkylène en C₂-C₃)-Si(R₆)2-; ou bien R₁ signifie un reste d'un énol, d'une énamine ou d'une ène-hydrazine ayant jusqu'à 20 atomes de carbone, lié par l'atome d'oxygène ou d'azote;
R₂ représente un indényle ou un cyclopentadiényle substitué éventuellement par des alkyle en C₁-C₆, alcényle en C₂-C₆, alcoxy en C₁-C₆, cycloalkyle en C₅ ou C₆, aryle en C₆-C₁₂, aralkyle en C₇-C₁₃, tri-(alcoxy en C₁-C₆) silyle, tri-(alkyl en C₁-C₆)silyle, un R₆ SO₂-ou un halogène,
Y représente un C, un Si ou un -Si-O-SiR₃R₄-,
R₃ et R₄, indépendemment l'un de l'autre, signifient un H, un alkyle en C₁-C₁₈, linéaire ou ramifié, un alcényle en C₂-C₁₂, un cycloalkyle en C₃-C₈, éventuellement substitué par des alkyles en C₁-C₄, ou cycloalcényle en C₃-C₈, ou un aryle en C₆-C₁₂, un aralkyle en C₇-C₁₆, un alkaryle en C₇-C₁₆, un alkaralkyle en C₈-C₁₆, un aralcényle en C₈-C₁₆ ou alkaralcényle en C₉-C₁₆ ou bien R₃ et R₄ ensemble signifient un -CₙH₂ₙ- avec n valant de 3 à 7, qui sont non-substitués ou substitués par les -CN, -F, -Cl, -Br, -nitro, alkylthio en C₁-C₁₂ ou alcoxy en C₁-C₁₂,
R₅ représente un hetéroaryle, un benzyle, un naphtylméthyle, un hetéroarylalkyle ou un aryle C₆-C₁₀, non substitué ou substitué par des alkyle en C₁-C₁₂, alcoxy en C₁-C₁₂, alkylthio en C₁-C₁₂, cyano, di-(alkyl en C₁-C₄)amino, di(alkyl en C₁-C₄)aminocarbonyle, fluor, aryle en C₆-C₁₀, R₆SO₂-, alcoxyméthyle en C₁-C₁₂, alkylthiométhyle en C₁-C₁₂, di(alkyl en C₁-C₄)aminométhyle ou un di(alkyl en C₁-C₄)aminocarbonylméthyle, l'hétéroaryle étant à 5 ou 6 chaînons et contenant un hétéroatome parmi le groupe O, S et N, ou signifie un alkyle en C₁-C₁₂, un alcényle ou un alcynyle en C₂-C₁₂, un cycloalkyle ou un cycloalcényle en C₃-C₈ et C(1) et C(2) sont des atomes de carbone chiraux, les composés de formule 1 se trouvant sur la forme prédominante d'un énantiomère ou d'un diastéréoisomère.

2. Composés selon la revendication 1, dans lesquels R₁ est un alkaralcynyle en C₉-C₁₆, un aralcynyle en C₈-C₁₆, un alkaralcényle en C₉-C₁₆, un aralcényle en C₈-C₁₆, un alkaralkyle en C₈-C₁₆, un alkaryle ou aralkyle en C₇-C₁₆, un aryle en C₆-C₁₂, un cycloalkyle ou cycloalcényle ayant de 3 à 8 atomes de carbone dans le cycle, un alcynyle en C₂-C₁₂, un alcényle en C₂-C₁₂, un alkyle en C₁-C₁₂, linéaire ou ramifié, substitué éventuellement une ou plusieurs fois par un R₁₈R₁₉N-, un cyano, un nitro, un alkylthio en C₁-C₆, un alcoxy en C₁-C₆ ou un -COR₇, dans lequel R₇ est un alcoxy en C₁-C₁₂; ou R₁ est un reste d'un énol, d'une énamine ou d'une ène-hydrazine ayant jusqu'à 20 atomes de carbone, lié par l'atome d'oxygène de l'énol ou par l'atome d'azote de l'énamine.

3. Composés selon la revendication 2, dans lesquels R₁ signifie un (alkyl en C₁-C₇)phénylpropargyle, un (alkyl en C₁-C₈)phényléthynyle, un (alkyl en C₁-C₈)-phénylvinyle, un phényléthynyle ou phénylpropargyle, un phénylvinyle, un (alkyl en C₁-C₈)phényl(alkyle en C₁-C₂), un phényl(alkyle en C₁-C₂), un (alkyl en C₁-C₁₀)phényle, un phényle, un cycloalkyle ou cycloalcényle avec de 3 à 6 atomes de carbone dans le cycle, un alcynyle en C₂-C₆, un alcényle en C₂-C₆, un alkyle en C₁-C₆, linéaire ou ramifié, substitué éventuellement une ou plusieurs fois par un R₁₈R₁₉N-, un cyano, un nitro, un alcoxy en C₁-C₆, un alkylthio en C₁-C₆ ou -COR₇, dans lequel R₇ est un alcoxy en C₁-C₁₂; ou R₁ signifie un reste d'un énol, d'une énamine ou d'une ène-hydrazine ayant jusqu'à 20 atomes de carbone, lié par l'atome d'oxygène de l'énol ou par l'atome d'azote de l'énamine.

4. Composés selon la revendication 2, dans lesquels R1 signifie un diméthylphényléthynyle ou diméthylphénylpropargyle, un méthylphényléthynyle, un phénylpropargyle, un phényléthynyle, un méthylphénylvinyle, un phénylvinyle, un méthylbenzyle, un 1-phényléth-2-yle, un benzyle, un méthylphényle, un phényle, un cyclopentényle et cyclohexényle, un cyclohexyle, un cyclopentyle, un propargyle, un éthynyle, un crotyle, un allyle, un vinyle, un éthyle, un méthyle, substitué éventuellement une ou plusieurs fois par un R₁₈R₁₉N-, un cyano, un nitro, un alcoxy en C₁-C₆, un alkylthio en C₁-C₆ ou -COR₇, dans lequel R₇ est un alcoxy en C₁-C₁₂; ou R₁ signifie un reste d'un énol, d'une énamine ou d'une ène-hydrazine ayant de 2 à 16 atomes de carbone, lié par l'atome d'oxygène de l'énol ou par l'atome d'azote de l'énamine.

5. Composés selon la revendication 1, dans lesquels R2 représente un indényle ou cyclopentadiényle substitué éventuellement par les alkyle en C₁-C₆, alcényle en C₂-C₆, alcoxy en C₁-C₆, cycloalkyle ayant de 5 ou 6 atomes de carbone dans le cycle, phényle, benzyle, trialcoxysilyle ayant de 1 à 6 atomes de carbone dans le groupe alcoxy, trialkylsilyle ayant de 1 à 6 atomes de carbone dans le groupe alkyle, F, Cl, Br, ou R₆SO₂- avec R₆ identique à un phényle, un tolyle, un benzyle ou un alkyle en C₁-C₆.

6. Composés selon la revendication 5, dans lesquels R2 représente un cyclopentadiényle substitué éventuellement par les alkyle en C₁-C₆ ou triméthylsilyle.

7. Composés selon la revendication 1, dans lesquels R₃ et R₄, indépendemment l'un de l'autre, représente un H, un alkyle en C₁-C₁₂, un alcényle en C₂-C₁₂, un cycloalkyle ou cycloalcényle ayant de 5 ou 6 atomes de carbone dans le cycle, éventuellement substitué par les alkyles en C₁-C₄, un phényle, un naphtyle, un benzyle, un alkylphényle en C₁-C₆ ou un alkylbenzyle en C₁-C₆ ou bien R₃ et R₄ représentent ensemble un -CₙH₂ₙ- avec n valant de 4 à 6, qui sont non substitués ou substitués par les -CN, -F, -Cl, -nitro, alkylthio en C₁-C₆ ou alcoxy en C₁-C₆.

8. Composés selon la revendication 7, dans lesquels R₃ et R₄, indépendemment l'un de l'autre, représentent un H, un alkyle en C₁-C₆, un cyclopentyle ou cyclohexyle éventuellement substitué par un alkyle en C₁-C₄, un phényle, un benzyle, un alkylphényle en C₁-C₆ ou un alkylbenzyle en C₁-C₆ ou bien R₃ et R₄ représentent ensemble un pentaméthylène ou un tétraméthylène.

9. Composés selon la revendication 1, dans lesquels R₃ est un H et R₄ ou R₃ et R₄ sont différents de H.

10. Composés selon la revendication 1, dans lesquels Y se trouve être un carbone.

11. Composés selon la revendication 1, dans lesquels R5 est un alcényle en C₂-C₄, un 2-furyle ou un aryle en C₆-C₁₀, qui est non substitué ou est substitué par les alkyle en C₁-C₆ ou F.

12. Composés selon la revendication 11, dans lesquels l'aryle est un phényle, un méthylphényle, un pentafluorophényle ou un naphtyle.

13. Composés selon la revendication 11, dans lesquels R5 est un propényle, un phényle, un pentafluorophényle, un méthylphényle ou un furyle.

14. Composés selon la revendication 1, dans lesquels
R1 représente un alkylbenzyle en C₁-C₆, ou un alkylphényle en C₁-C₆, un benzyle, un phényle, un cycloalkyle ou cycloalcényle avec de 3 à 6 atomes de carbone dans le cycle, substitué éventuellement par des alkyles en C₁-C₄, un alcényle en C₂-C₆, un alkyle en C₁-C₄, linéaire ou ramifié, lequel est non substitué ou substitué par les amino secondaire, cyano, nitro, alcoxy en C₁-C₆, alkylthio en C₁-C₆ ou -COR₇, dans lequel R7 est un alcoxy en C₁-C₁₂, ou,
R1 signifie un reste d'énol, d'une énamine ou d'une ène-hydrazine ayant jusqu'à 12 atomes de carbone, lié par un atome d'oxygène de l'énol ou un atome d'azote de l'énamine,
R2 est un cyclopentadiènyle, qui est non substitué ou est substitué par les alkyle en C₁-C₄ ou triméthylsilyle,
Y représente un carbone,
R₃ est un H ou a la signification de R₄ et
R₄ représente un alkyle en C₁-C₁₂, un phényle, un benzyle, un cyclopentyle, un cyclohexyle ou,
R₃ et R₄ signifient ensemble un tétra ou pentaméthylène,
R₅ représente un alcényle en C₂-C₄, un phényle, un pentafluorophényle ou un furyle, et
Me signifie un titane, un zirconium ou un hafnium tétravalant.

15. Composés selon la revendication 1, dans lesquels les atomes C(1) et C(2) de la formule 1 ont soit la configuration R,R soit la configuration S,S.

16. Composés selon la revendication 1, dans lesquels Y est un atome de carbone chiral, sous la forme de diastéréoisomères.

17. Composés selon la revendication 1, dans lesquels R1 est un alkyle en C₁-C₄, un allyle ou le reste d'un énolate d'ester ayant de 2 à 12 atomes de carbone qui peut être substitué par R₁₈R₁₉N-, R₂ signifie un cyclopentadiènyle substitué éventuellement par les méthyle ou triméthylsilyle, R3 représente un H ou a la signification R4 et R4 est un alkyle en C₁-C₆, R₅ signifie un propényle, phényle, un pentafluorophényle ou un furyle et Me se trouve être un titane, un zirconium ou un hafnium tétravalent et Y représente un carbone.

18. Procédé de préparation des composés de formule I selon la revendication 1, caractérisé en ce qu'on fait réagir un composé de formule II 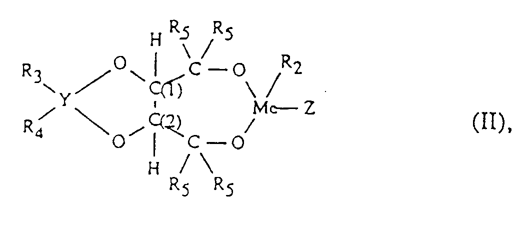 avec un composé de formule III
R₁^{⊖}-M^{⊕}
en présence d'un solvant inerte et d'un gaz protecteur, R₁, R₂, R₃, R₄, R₅, Me et Y ayant les significations données dans la revendication 1, Z représentant un anion et M^{⊕} représentant un Li^{⊕}, un Na^{⊕}, un K^{⊕}, un MgX^{⊕}, un ZnX^{⊕}, un CdX^{⊕}, un HgX^{⊕}, un CuX^{⊕}, ou un ammonium quaternaire, X signifiant un halogène.

19. Composés de formule II, 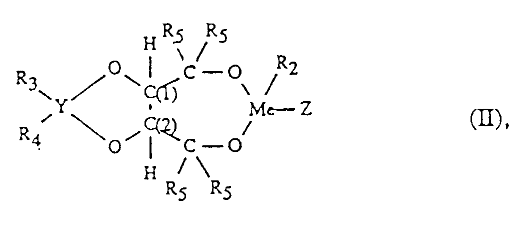 dans laquelle R₂, R₃, R₄, R₅, Y et Me ont les significations données dans la revendication 1 et Z représente un anion.

20. Utilisation de composés de formule I selon la revendication 1 en tant que réactifs énantiosélectifs pour des composés ayant des groupes aldéhyde, cétone et/ou imine N-substituée.

21. Procédé de préparation d'alcools secondaires et tertiaires et d'amines secondaires, caractérisé en qu'on met en réaction un aldéhyde, une cétone, une aldimine N-substituée ou une cétimine avec 1 mole d'un composé de formule I selon la revendication 1 par mole de groupe aldéhyde, cétone ou imine.

22. Procédé selon la revendication 21, caractérisé en ce qu'il est réalisé une température comprise entre -80 et 30°C.

## Revendications (Revendications pour l'(les) Etat(s) contractant(s) suivant(s): ES)

1. Procédé de préparation des composés de formule I 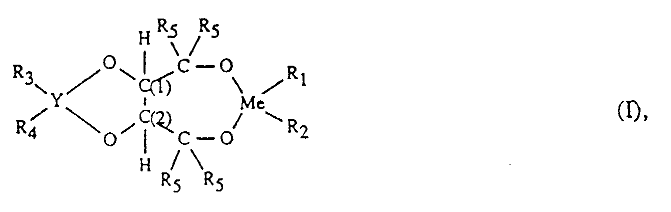 dans laquelle
a) Me représente un titane tétravalent et R₁ représente les allyle, 1-méthylallyle, 2-méthylallyle -CH₂CH=CH-P(C₆H₅), -CH(SO₂R₆)CH=CH-Si(R₆)₃, 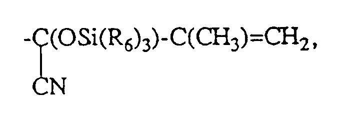 -CH₂-CH=CH-SO₂-R₆, -CH₂-CH=CH-OR' ou 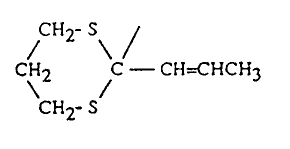 R₆, se trouve être un phényle, un benzyle ou un alkyle en C₁-C₈ et R' signifie un alkyle en C₁-C₈ ou un -Si(R₆)₃, ou R₁ est un reste d'un énol, d'une énamine ou d'une ène-hydrazine ayant jusqu'à 20 atomes de carbone et étant lié par l'atome d'oxygène ou d'azote; ou
b) Me représente un zirconium ou un hafnium tétravalent et R₁ signifie un alkyle en C₁-C₁₈ linéaire ou ramifié, un alcényle en C₂-C₁₂, un alcynyle en C₂-C₁₂, ou un cycloalkyle en C₃-C₈ éventuellement substitué par des alkyle en C₁-C₄ ou un cycloalcényle en C₃-C₈, ou un aryle en C₆-C₁₂, un alkaryle en C₇-C₁₆, un aralkyle en C₆-C₁₆, un alkaralkyle en C₈-C₁₆ un aralcényle en C₈-C₁₆, un alkaralcényle en C₉-C₁₆, un aralcynyle en C₈-C₁₆ ou un alkaralcynyle en C₉-C₁₆, qui sont non substitués ou substitués une plusieurs fois par les (C₆H₅)₂P-, (R₆O)₂P(O)-, (R₆)₃Si-, (R₆)₃SiO-, R₆SO₂-, -S-(alkylène en C₂-C₄)-S-, -O-(alkylène en C₂-C₄)-O-, R₆ représentant un phényle, un benzyle ou un alkyle en C₁-C₈, cyano, F, nitro, alkylthio en C₁-C₁₂, alcoxy en C₁-C₁₂, -NR₁₈R₁₉ ou -COR₇, R₇ étant un alcoxy en C₁-C₁₂, R₁₈ et R₁₉ signifiant un alkyle en C₁-C₁₂, un cyclopentyle, un cyclohexyle, un phényle, un benzyle ou (R₆)₃Si ou R₁₈ et R₁₉ ensemble signifient un alkylène en C₃-C₆, 3-oxapentylène ou un -Si(R₆)₂-(alkylène en C₂-C₃)-Si(R₆)₂-; ou bien R₁ signifie un reste d'un énol, d'une énamine ou d'une ène-hydrazine ayant jusqu'à 20 atomes de carbone, lié sur l'atome d'oxygène ou d'azote;
R₂ représente un indényle ou un cyclopentadiényle substitué éventuellement par des alkyle en C₁-C₆, alcényle en C₂-C₆, alcoxy en C₁-C₆, cycloalkyle en C₅ ou C₆, aryle en C₆-C₁₂ aralkyle en C₇-C₁₃, tri-(alcoxy en C₁-C₆) silyle, tri-(alkyl en C₁-C₆) silyle, un R₆ SO₂-ou un halogène,
Y représente un C, un Si ou un -Si-O-SiR₃R₄-,
R₃ et R₄, indépendemment l'un de l'autre, signifient un H, un alkyle en C₁-C₁₈, linéaire ou ramifié, un alcényle en C₂-C₁₂, un cycloalkyle en C₃-C₈, éventuellement substitué par des alkyles en C₁-C₄, ou cycloalcényle en C₃-C₈, ou un aryle en C₆-C₁₂, un aralkyle en C₇-C₁₆, un alkaryle en C₇-C₁₆, un alkaralkyle en C₈-C₁₆, un aralcényle en C₈-C₁₆ ou alkaralcényle en C₉-C₁₆ ou bien R₃ et R₄ ensemble signifient un -CₙH₂ₙ- avec n valant de 3 à 7, qui sont non-substitués ou substitués par les -CN, -F, -Cl, -Br, -nitro, alkylthio en C₁-C₁₂ ou alcoxy en C₁-C₁₂,
R₅ représente un hetéroaryle, un benzyle, un naphtylméthyle, un hetéroarylalkyle ou un aryle C₆-C₁₀, non substitué ou substitué par des alkyle en C₁-C₁₂, alcoxy en C₁-C₁₂, alkylthio en C₁-C₁₂, cyano, di-(alkyl en C₁-C₄)amino, di(alkyl en C₁-C₄)aminocarbonyle, fluor, aryle en C₆-C₁₀, R₆SO₂-, alcoxyméthyle en C₁-C₁₂, alkylthiométhyle en C₁-C₁₂, di(alkyl en C₁-C₄)aminométhyle ou un di(alkyl en C₁-C₄)aminocarbonylméthyle, l'hétéroaryle étant à 5 ou 6 chaînons et contenant un hétéroatome parmi le groupe O, S et N, ou signifie un alkyle en C₁-C₁₂, un alcényle ou un alcynyle en C₂-C₁₂, un cycloalkyle ou un cycloalcényle en C₃-C₈ et C(1) et C(2) sont des atomes de carbone chiraux, les composés de formule 1 se trouvant sous la forme prédominante d'un énantiomère ou d'un diastéréoisomère, caractérisé en ce qu'on fait réagir un composé de formule II 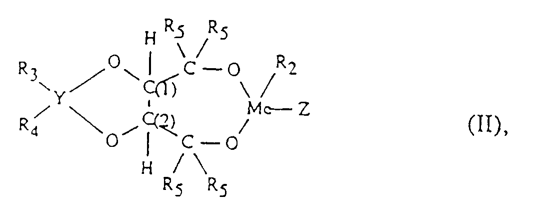 avec un composé de formule III
R₁^{⊖}-M^{⊕}
en présence d'un solvant inerte et d'un gaz protecteur, R₁, R₂, R₃, R₄, R₅, Me et Y ayant les significations données ci-dessus, Z représentant un anion et M^{⊕} représentant un Li^{⊕}, un Na^{⊕}, un K^{⊕}, un MgX^{⊕}, un ZnX^{⊕}, un CdX^{⊕}, un HgX^{⊕}, un CuX^{⊕}, ou un ammonium quaternaire, X signifiant un halogène.

2. Procédé selon la revendication 1, dans lequel R₁ est un alkaralcynyle en C₉-C₁₆, un aralcynyle en C₈-C₁₆, un alkaralcényle en C₉-C₁₆, un aralcényle en C₈-C₁₆, un alkaralkyle en C₈-C₁₆, un alkaryle ou aralkyle en C₇-C₁₆, un aryle en C₆-C₁₂, un cycloalkyle ou cycloalcényle ayant de 3 à 8 atomes de carbone dans le cycle, un alcynyle en C₂-C₁₂, un alcényle en C₂-C₁₂, un alkyle en C₁-C₁₂, linéaire ou ramifié, substitué éventuellement une ou plusieurs fois par un R₁₈R₁₉N-, un cyano, un nitro, un alkylthio en C₁-C₆, un alcoxy en C₁-C₆ ou un -COR₇, dans lequel R₇ est un alcoxy en C₁-C₁₂; ou R₁ est un reste d'un énol, d'une énamine ou d'une ène-hydrazine ayant jusqu'à 20 atomes de carbone, lié par l'atome d'oxygène de l'énol ou par l'atome d'azote de l'énamine.

3. Procédé selon la revendication 2, dans lequel R₁ signifie un (alkyl en C₁-C₇)phénylpropargyle, un (alkyl en C₁-C₈)phényléthynyle, un (alkyl en C₁-C₈)phénylvinyle, un phényléthynyle ou phénylpropargyle, un phénylvinyle, un (alkyl en C₁-C₈)phényl(alkyle en C₁-C₂), un phényl(alkyle en C₁-C₂), un (alkyl en C₁-C₁₀)phényle, un phényle, un cycloalkyle ou cycloalcényle avec de 3 à 6 atomes de carbone dans le cycle, un alcynyle en C₂-C₆, un alcényle en C₂-C₆, un alkyle en C₁-C₆, linéaire ou ramifié, substitué éventuellement une ou plusieurs fois par un R₁₈R₁₉N-, un cyano, un nitro, un alcoxy en C₁-C₆, un alkylthio en C₁-C₆ ou -COR₇, dans lequel R₇ est un alcoxy en C₁-C₁₂; ou R₁ signifie un reste d'un énol, d'une énamine ou d'une ène-hydrazine ayant jusqu'à 20 atomes de carbone, lié par l'atome d'oxygène de l'énol ou par l'atome d'azote de l'énamine.

4. Procédé selon la revendication 2, dans lequel R1 signifie un diméthylphényléthynyle ou diméthylphénylpropargyle, un méthylphényléthynyle, un phénylpropargyle, un phényléthynyle, un méthylphénylvinyle, un phénylvinyle, un méthylbenzyle, un 1-phényléth-2-yle, un benzyle, un méthylphényle, un phényle, un cyclopentényle et cyclohexényle, un cyclohexyle, un cyclopentyle, un propargyle, un éthynyle, un crotyle, un allyle, un vinyle, un éthyle, un méthyle, substitué éventuellement une ou plusieurs fois par un R₁₈R₁₉N-, un cyano, un nitro, un alcoxy en C₁-C₆, un alkylthio en C₁-C₆ ou -COR₇, dans lequel R₇ est un alcoxy en C₁-C₁₂; ou R₁ signifie un reste d'un énol, d'une énamine ou d'une ène-hydrazine ayant de 2 à 16 atomes de carbone, lié par l'atome d'oxygène de l'énol ou par l'atome d'azote de l'énamine.

5. Procédé selon la revendication 1, dans lequel R2 représente un indényle ou cyclopentadiényle substitué éventuellement par les alkyle en C₁-C₆, alcényle en C₂-C₆, alcoxy en C₁-C₆, cycloalkyle ayant de 5 ou 6 atomes de carbone dans le cycle, phényle, benzyle, trialcoxysilyle ayant de 1 à 6 atomes de carbone dans le groupe alcoxy, trialkylsilyle ayant de 1 à 6 atomes de carbone dans le groupe alkyle, F, Cl, Br, ou R₆SO₂- avec R₆ identique à un phényle, un tolyle, un benzyle ou un alkyle en C₁-C₆.

6. Procédé selon la revendication 5, dans lequel R2 représente un cyclopentadiényle substitué éventuellement par les alkyle en C₁-C₆ ou triméthylsilyle.

7. Procédé selon la revendication 1, dans lequel R₃ et R₄, indépendemment l'un de l'autre, représente un H, un alkyle en C₁-C₁₂, un alcényle en C₂-C₁₂, un cycloalkyle ou cycloalcényle ayant de 5 ou 6 atomes de carbone dans le cycle, éventuellement substitué par les alkyles en C₁-C₄, un phényle, un naphtyle, un benzyle, un alkylphényle en C₁-C₆ ou un alkylbenzyle en C₁-C₆ ou bien R₃ et R₄ représentent ensemble un -CₙH₂ₙ- avec n valant de 4 à 6, qui sont non substitués ou substitués par les -CN, F, -Cl, -nitro, alkylthio en C₁-C₆ ou alcoxy en C₁-C₆.

8. Procédé selon la revendication 7, dans lequel R₃ et R₄, indépendemment l'un de l'autre, représentent un H, un alkyle en C₁-C₆, un cyclopentyle ou cyclohexyle éventuellement substitué par un alkyle en C₁-C₄, un phényle, un benzyle, un alkylphényle en C₁-C₆ ou un alkylbenzyle en C₁-C₆ ou bien R₃ et R₄ représentent ensemble un pentaméthylène ou un tétraméthylène.

9. Procédé selon la revendication 1, dans lequel R₃ est un H et R₄ ou R₃ et R₄ sont différents de H.

10. Procédé selon la revendication 1, dans lequel Y se trouve être un carbone.

11. Procédé selon la revendication 1, dans lequel R5 est un alcényle en C₂-C₄, un 2-furyle ou un aryle en C₆-C₁₀, qui est non substitué ou est substitué par les alkyle en C₁-C₆ ou F.

12. Procédé selon la revendication 11, dans lequel l'aryle est un phényle, un méthylphényle, un pentafluorophényle ou un naphtyle.

13. Procédé selon la revendication 11, dans lequel R5 est un propényle, un phényle, un pentafluorophényle, un méthylphényle ou un furyle.

14. Procédé selon la revendication 1, dans lequel
R1 représente un alkylbenzyle en C₁-C₆, ou un alkylphényle en C₁-C₆, un benzyle, un phényle, un cycloalkyle ou cycloalcényle avec de 3 à 6 atomes de carbone dans le cycle, substitué éventuellement par des alkyles en C₁-C₄, un alcényle en C₂-C₆, un alkyle en C₁-C₄, linéaire ou ramifié, lequel est non substitué ou substitué par les amino secondaire, cyano, nitro, alcoxy en C₁-C₆, alkylthio en C₁-C₆ ou -COR₇, dans lequel R7 est un alcoxy en C₁-C₁₂, ou,
R1 signifie un reste d'énol, d'une énamine ou d'une ène-hydrazine ayant jusqu'à 12 atomes de carbone, lié par un atome d'oxygène de l'énol ou un atome d'azote de l'énamine,
R2 est un cyclopentadiènyle, qui est non substitué ou est substitué par les alkyle en C₁-C₄ ou triméthylsilyle,
Y représente un carbone,
R₃ est un H ou a la signification de R₄ et
R₄ représente un alkyle en C₁-C₁₂, un phényle, un benzyle, un cyclopentyle, un cyclohexyle ou,
R₃ et R₄ signifient ensemble un tétra ou pentaméthylène,
R₅ représente un alcényle en C₂-C₄, un phényle, un pentafluorophényle ou un furyle, et
Me signifie un titane, un zirconium ou un hafnium tétravalant.

15. Procédé selon la revendication 1, dans lequel les atomes C(1) et C(2) de la formule 1 ont soit la configuration R,R soit la configuration S,S.

16. Procédé selon la revendication 1, dans lequel Y est un atome de carbone chiral, sous la forme de diastéréoisomères.

17. Procédé selon la revendication 1, dans lequel R1 est un alkyle en C₁-C₄, un allyle ou le reste d'un énolate d'ester ayant de 2 à 12 atomes de carbone qui peut être substitué par R₁₈R₁₉N-, R₂ signifie un cyclopentadiènyle substitué éventuellement par les méthyle ou triméthylsilyle, R3 représente un H ou a la signification R4 et R4 est un alkyle en C₁-C₆, R₅ signifie un propényle, phényle, un pentafluorophényle ou un furyle et Me se trouve être un titane, un zirconium ou un hafnium tétravalant et Y se trouve être un carbone

18. Procédé de préparation des composés de formule I selon la revendication 1, caractérisé en ce qu'on fait réagir un composé de formule II 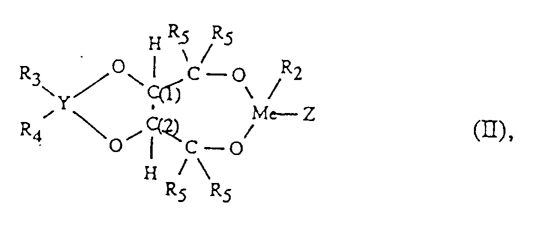 avec un composé de formule III
R₁^{⊖}-M^{⊕}
en présence d'un solvant inerte et d'un gaz protecteur, R₁, R₂, R₃, R₄, R₅, Me et Y ayant les significations données dans la revendication 1, Z représentant un anion et M^{⊕} représentant un Li^{⊕}, un Na^{⊕}, un K^{⊕}, un MgX^{⊕}, un ZnX^{⊕}, un CdX^{⊕}, un HgX^{⊕}, un CuX^{⊕}, ou un ammonium quaternaire, X signifiant un halogène.

19. Utilisation de composés de formule I selon la revendication 1 en tant que réactifs énantiosélectifs pour des composés ayant des groupes aldéhyde, cétone et/ou imine N-substituée.

20. Procédé de préparation d'alcools secondaires et tertiaires et d'amines secondaires, caractérisé en qu'on met en réaction un aldéhyde, une cétone, une aldimine N-substituée ou une cétimine avec 1 mole d'un composé de formule I selon la revendication 1 par mole de groupe aldéhyde, cétone ou imine.

21. Procédé selon la revendication 20, caractérisé en ce qu'il est réalisé une température comprise entre -80 et 30°C.
